# EUROPEAN PATENT SPECIFICATION

(11) **EP 3 440 109 B1**
(45) Date of publication and mention of the grant of the patent: **23.07.2025**
(21) Application number: 17711664.7
(22) Date of filing: 17.03.2017
(51) Int. Cl.: A61P 35/00, C07K 16/28, A61K 39/00

(54) **ANTI CD25 FC GAMMA RECEPTOR ANTIBODIES FOR TUMOR SPECIFIC CELL DEPLETION IN COMBINATION WITH PD-1 ANTAGONISTS**
ANTICORPS ANTI-CD25 RECEPTEUR FC GAMMA AUX FINS DE REDUCTION SPECIFIQUE DE CELLULES TUMORALES AVEC PD-1 ANTAGONISTE
ANTI CD25 FC GAMMA REZEPTOR ANTIKORPER ZUR TUMORZELLDEPLETION MIT PD-1 ANTAGONISTEN

(30) Priority: 07.04.2016 GB 201605947
(43) Date of publication of application: 13.02.2019
(73) Proprietor: Cancer Research Technology Limited, London E20 1JQ (GB)
(72) Inventor: QUEZADA, Sergio, London EC1V 4AD (GB); PEGGS, Karl, London EC1V 4AD (GB); ARCE VARGAS, Frederick, London EC1V 4AD (GB)
(74) Representative: Leonard, Thomas Charles
(86) International application number: PCT/EP2017/056469
(87) International publication number: WO 2017/174331

(56) References cited:
- WO-A1-2014/145907
- RECH A J ET AL: "Clinical use of anti-CD25 antibody daclizumab to enhance immune responses to tumor antigen vaccination by targeting regulatory T cells", ANNALS OF THE NEW YORK ACADEMY OF SCIENCES, NEW YORK ACADEMY OF SCIENCES, US, vol. 1174, 1 September 2009 (2009-09-01), pages 99 - 106, XP002634041, ISSN: 0077-8923, [retrieved on 20090914], DOI: 10.1111/J.1749-6632.2009.04939.X
- SUTMULLER R P M ET AL: "SYNERGISM OF CYTOTOXIC T LYMPHOCYTE-ASSOCIATED ANTIGEN 4 BLOCKADE AND DEPLETION OF CD25+ REGULATORY T CELLS IN ANTITUMOR THERAPY REVEALS ALTERNATIVE PATHWAYS FOR SUPPRESSION OF AUTOREACTIVE CYTOTOXIC T LYMPHOCYTE RESPONSES", JOURNAL OF EXPERIMENTAL MEDICINE, ROCKEFELLER UNIVERSITY PRESS, US, vol. 194, no. 6, 17 September 2001 (2001-09-17), pages 823 - 832, XP008040742, ISSN: 0022-1007, DOI: 10.1084/JEM.194.6.823
- P. YU ET AL: "Simultaneous inhibition of two regulatory T-cell subsets enhanced Interleukin-15 efficacy in a prostate tumor model", PROCEEDINGS OF THE NATIONAL ACADEMY OF SCIENCES, vol. 109, no. 16, 17 April 2012 (2012-04-17), pages 6187 - 6192, XP055147800, ISSN: 0027-8424, DOI: 10.1073/pnas.1203479109
- MIKAYEL MKRTICHYAN ET AL: "Anti-PD-1 synergizes with cyclophosphamide to induce potent anti-tumor vaccine effects through novel mechanisms", EUROPEAN JOURNAL OF IMMUNOLOGY, WILEY-VCH, HOBOKEN, USA, vol. 41, no. 10, 17 August 2011 (2011-08-17), pages 2977 - 2986, XP071225170, ISSN: 0014-2980, DOI: 10.1002/EJI.201141639
- NIMMERJAHN FALK ET AL: "Fc[gamma]R dependent mechanisms of cytotoxic, agonistic, and neutralizing antibody activities", TRENDS IN IMMUNOLOGY, ELSEVIER LTD. * TRENDS JOURNALS, GB, vol. 36, no. 6, 13 May 2015 (2015-05-13), pages 325 - 336, XP029611604, ISSN: 1471-4906, DOI: 10.1016/J.IT.2015.04.005
- FURNESS ANDREW J S ET AL: "Impact of tumour microenvironment and Fc receptors on the activity of immunomodulatory antibodies", TRENDS IN IMMUNOLOGY, ELSEVIER LTD. * TRENDS JOURNALS, UNITED KINGDOM, vol. 35, no. 7, July 2014 (2014-07-01), pages 290 - 298, XP002750996, ISSN: 1471-4981, DOI: 10.1016/J.IT.2014.05.002

## Description

### FIELD OF THE INVENTION

The present invention is in the field of cancer immunotherapy, and relates to treating a solid tumour, wherein the treatment involves the use of an antibody to CD25.

### BACKGROUND OF THE INVENTION

Cancer immunotherapy involves the use of a subject's own immune system to treat or prevent cancer. Immunotherapies exploit the fact that cancer cells often have subtly different molecules on their surface that can be detected by the immune system. These molecules, or cancer antigens, are most commonly proteins, but also include molecules such as carbohydrates. Immunotherapy thus involves provocation of the immune system into attacking tumour cells via these target antigens. However, malignant tumours, in particular solid tumours, can escape immune surveillance by means of various mechanisms both intrinsic to the tumour cell and mediated by components of the tumour microenvironment. Amongst the latter, tumour infiltration by regulatory T cells (Treg cells or Tregs) and, more specifically, an unfavorable balance of effector T cells (Teff) versus Tregs (i.e. a low ratio of Teff to Treg), have been proposed as critical factors (Smyth M et al., 2014, Immunol Cell Biol. 92, 473-4).

Since their discovery, Tregs have been found to be critical in mediating immune homeostasis and promoting the establishment and maintenance of peripheral tolerance. However, in the context of cancer their role is more complex. As cancer cells express both self- and tumour-associated antigens, the presence of Tregs, which seek to dampen effector cell responses, can contribute to tumour progression. The infiltration of Tregs in established tumours therefore represents one of the main obstacles to effective anti-tumour responses and to treatment of cancers in general. Suppression mechanisms employed by Tregs are thought to contribute significantly to the limitation or even failure of current therapies, in particular immunotherapies that rely on induction or potentiation of anti-tumour responses (Onishi H et al;, 2012 Anticanc. Res. 32, 997-1003).

Depletion of Tregs as therapeutic approach for treating cancer is an approach that is supported by studies having shown the contribution of Tregs to tumour establishment and progression in murine models. Moreover, tumour infiltration by Tregs has also been associated with worse prognosis in several human cancers (Shang B et al., 2015, Sci Rep. 5:15179). However, depletion of Tregs in tumours is complex, and results of studies in this area have been discrepant. Thus, there is a need in the art for a method of treating cancer involving depletion of Tregs.

Among the potential molecular targets for achieving depletion of Tregs, the IL-2/CD25 interaction has been object of several studies in murine models, some of them involving the use of PC61, a rat anti-mouse CD25 antibody (Setiady Y et al., 2010. Eur J Immunol. 40: 780-6). The CD25 binding and functional activities of this antibody have been compared to those of panel of monoclonal antibodies generated by different authors (Lowenthal J.W et al., 1985. J. Immunol., 135, 3988-3994; Moreau, J.-L et al., 1987. Eur. J. Immunol. 17,929-935; Volk HD et al., 1989 Clin. exp. Immunol. 76, 121-5; Dantal J et al., 1991, Transplantation 52:110-5).

In this manner, three epitopes for anti-mouse CD25 within such target that are distinct or common from the mouse IL-2 binding site have been characterized. PC61 (having mouse IgG1 isotype) blocks or inhibits the binding of IL-2 to CD25, as many other hybridomas for anti-mouse CD25 antibodies (and most of those disclosed as anti-human CD25 antibodies; see for instance WO2004/045512, WO2006/108670, WO1993/011238, and WO1990/007861). Moreover, the binding of PC61 to mouse CD25 is not affected, as for other anti-mouse CD25 antibodies such as 7D4, by ADP-ribosylation of CD25 in the IL-2 binding site (Teege S et al., 2015, Sci Rep 5: 8959).

Some literature refers to the use of anti-CD25, alone or in combination, in cancer or in connection to Treg depletion. (WO2004/074437; WO2006/108670; WO2006/050172; WO2011/077245; WO2016/021720; WO2004/045512; Grauer O et al., 2007 Int. J. Cancer: 121: 95-105). However, when tested in mouse models of cancer, the rat anti-mouse CD25 PC61 failed to demonstrate anti-tumour activity when delivered after tumour establishment.

Sutmuller RP et al, 2001, J Exp Med 194(6):823 discloses the use of an anti-CD25 antibody (PC61) in combination with an anti-CTLA-4 antibody. Mkrtichyan M et al, 2011, Eur J Immunol, 41:2977, discloses the use of an anti-PD-1 antibody (CT-11) and cyclophosphamide in combination with a peptide vaccine.

In the context of a murine model of autoimmunity, the anti-CD25 PC61 antibody was re-engineered to evaluate the effect of an highly divergent Fc effector function within an anti-CD25 antibody on IL-2 receptor blocking and depletion of peripheral Treg (Huss D et al., 2016. Immunol. 148: 276-86). However, the ability to deplete Tregs in tumours, or to mediate anti-tumor therapeutic activity, had never been evaluated for PC61 (as such, as an engineered antibody, or as an anti-human CD25 designed or characterized as having CD25 binding features similar to those of PC61 for mouse CD25), alone or in combination with other antibodies or anti-cancer compounds.

### SUMMARY

The invention is set out in the appended set of claims.

The present invention provides anti-CD25 antibodies and uses of anti-CD25 antibodies that are characterized by structural elements that allow depleting efficiently Tregs, in particular within tumours. At this scope, the structural and functional features of rat IgG1 PC-61 (as described with respect to mouse CD25) have been modified in order to provide antibodies that present surprisingly improved features in terms of use as depleting Tregs and efficacy against tumours, alone or in combination with other anti-cancer agents. These findings can be used for defining and generating novel anti-human CD25 that provide comparable effects against tumours in human subjects.

Hence a key discovery by the inventors is the unexpected finding that anti mouse anti-CD25 PC61 is only able to deplete Treg in the lymph nodes and circulation whilst failing to do so within the tumour. Lack of Treg depletion in the tumour correlates with lack of anti-tumour activity. This new and unexpected data prompted the inventors to increase the depleting activity of anti mouse CD25 via Fc engeneering which lead to potent depletion of intra-tumoral Treg and anti-tumour activity.

Described is a method of treating a human subject who has cancer comprising the step of administering an anti-CD25 antibody to a subject, wherein said subject has a tumour (preferably a solid tumour), wherein said anti-CD25 antibody is an IgG1 antibody that binds to at least one activating Fcγ receptor (preferably selected from FcγRI, FcγRIIc, and FcγRIIIa) with high affinity, and depletes tumour-infiltrating regulatory T cells.

Such antibody preferably has a dissociation constant (K_{d}) for CD25 of less than 10⁻⁸ M and/or a dissociation constant for at least one activating Fcγ receptor of less than about 10⁻⁶ M. Most preferably, the anti-CD25 antibody is characterized by other features related to Fcγ receptors, in particular:
(a) binds to Fcγ receptors with an activatory to inhibitory ratio (A/I) superior to 1; and/or
(b) binds to at least one of FcγRI, FcγRIIc and FcγRIIIa with higher affinity than it binds to FcγRIIb.

Given the use of the anti-CD25 antibody in therapeutic methods, it can present further preferred features. The anti-CD25 antibody is preferably a monoclonal antibody, in particular a human or humanized antibody. Moreover, in view of its interactions with immune cells and/or other components of the component of the immune system for exerting its activities, the anti-CD25 antibody may further elicit an enhanced CDC, ADCC and/or ADCP response, preferably an increased ADCC and/or ADCP response, more preferably an increased ADCC response.

The anti-CD25 antibody (as generally defined above and in further details in the Detailed Description) can be used in methods of treating a human subject, wherein said anti-CD25 antibody is administered to a subject who has an established, solid tumour (preferably in a method further comprising the step of identifying a subject who has a solid tumour). Such methods may further comprise administering an immune checkpoint inhibitor to said subject, for example in the form of an antibody binding and inhibiting an immune checkpoint protein. A preferred immune checkpoint inhibitor is a PD-1 antagonist, which can be an anti-PD-1 antibody or an anti-PD-L1 antibody. More in general, an anti-CD25 antibody can be used in methods of depleting regulatory T cells in a solid tumour in a subject comprising the step of administering said anti-CD25 antibody to said subject.

The anti-CD25 antibody as described herein can be used for the manufacture of a medicament for the treatment of cancer in a human subject, wherein said subject has a solid tumour. At this scope, said antibody is for administration in combination with an immune checkpoint inhibitor, preferably a PD-1 antagonist.

Also described is a combination of an anti-CD25 antibody as defined above with another anti-cancer compound (preferably an immune checkpoint inhibitor or other compounds as indicated in the Detailed Description) for use in the treatment of cancer in a human subject, wherein said subject has a solid tumour and the anti-CD25 antibody and the anti-cancer compound (for example, an immune checkpoint inhibitor such a PD-1 antagonist) are administered simultaneously, separately or sequentially. At this scope also described is a kit for use in the treatment of cancer comprising an anti- CD25 antibody, as defined above, and an anti-cancer compound (for example, an immune checkpoint inhibitor such a PD-1 antagonist),

Also described is a pharmaceutical composition comprising an anti-CD25 antibody as defined above in a pharmaceutically acceptable medium. Such composition may also comprise an anti-cancer compound (for example, an immune checkpoint inhibitor such a PD-1 antagonist),

Further described is a bispecific antibody comprising:
(a) a first antigen binding moiety that binds to CD25; and
(b) a second antigen binding moiety that binds to another antigen;
wherein the bispecific antibody is an IgG1 antibody that binds to at least one activatory Fcγ receptor with high affinity and depletes tumour-infiltrating regulatory T cells. Preferably, such second antigen binding moiety binds to an antigen selected from an immune checkpoint protein, a tumour-associated antigen, or is (or is based on) an anti-human activatory Fc Receptor antibody (anti-FcγRI, anti-FcγRIIc, or anti-FcγRIIIa antibody) or is (or is based on) an antagonistic anti-human FcγRIIb antibody.

Preferably, such bispecific antibody comprises a second antigen binding moiety that binds an immune checkpoint protein that is selected from the group consisting of PD-1, CTLA-4, BTLA, KIR, LAG3, VISTA, TIGIT, TIM3, PD-L1, B7H3, B7H4, PD-L2, CD80, CD86, HVEM, LLT1, GAL9, GITR, OX40, CD137, and ICOS. Such immune checkpoint protein is preferably expressed on a tumour cell, and most preferably is selected from PD-1, PD-L1, and CTLA-4. The second antigen binding moiety that binds to an immune checkpoint protein can be comprised in or based on a commercially available antibody that acts as an immune checkpoint inhibitor, for example:
(a) in the case of PD-1, the anti-PD-1 antibody can be Nivolumab or Pembrolizumab.
(b) In the case of PD-L1, the anti-PD-L1 is Atezolizumab;
(c) In case of CTLA-4, the anti-CTLA-4 is Ipilimumab.

Such bispecfic antibody can be provided in any commercially available format, including Duobody, BiTE DART, CrossMab, Knobs-in-holes, Triomab, or other appropriate molecular format of bispecific antibody and fragments thereof.

Alternatively, such bispecific antibody comprises a second antigen binding moiety that binds to a tumour-associated antigen. In this alternative embodiment such antigens and corresponding antibodies include, without limitation CD22 (Blinatumomab), CD20 (Rituximab, Tositumomab), CD56 (Lorvotuzumab), CD66e/CEA (Labetuzumab), CD152/CTLA-4 (Ipilimumab), CD221/IGF1R (MK-0646), CD326/Epcam (Edrecolomab), CD340/HER2 (Trastuzumab, Pertuzumab), and EGFR (Cetuximab, Panitumumab).

The combination of anti-CD25 antibody with another anti-cancer compound, or the bispecific antibodies as defined above, can be used in a method of treating cancer, comprising the step of administering said combination or said bispecific antibody to a subject, in particular when the subject has a solid tumour, and for use in the treatment of cancer in a subject.

Further definitions of the anti-human CD25 antibody of the invention and of their uses in methods for treating cancer, in pharmaceutical compositions, in combinations with other anti-cancer compounds, in bispecific antibodies, are provided in the Detailed Description and in the Examples.

### DETAILED DESCRIPTION

Described herein is a method of treating or preventing cancer, in particular a solid tumour, in a subject, comprising the step of administering an antibody that binds to CD25 to said subject whereby the anti-CD25 antibodies are characterized by structural elements that allow depleting efficiently Tregs, in particular within tumours. Also described is an antibody that binds to CD25, as defined herein, for use in the treatment or prevention of cancer, in particular a solid tumour. Alternatively put, also described is the use of an antibody that binds to CD25 and that allows depleting efficiently Tregs for the manufacture of a medicament for the treatment or prevention of cancer, in particular a solid tumour. Also described is the use of an antibody that binds CD25 and that allows depleting efficiently Tregs in the treatment or prevention of cancer, in particular a solid tumour.

The present invention discloses how switching the isotype of an anti-CD25 antibody (exemplified by the rat anti-mouse CD25 antibody PC61) to a depleting isotype (mouse IgG2 for PC61, but equivalent to IgG1 in human) leads to improved depletion of regulatory T cells in a solid tumour context. Moreover, the present inventors have found for the first time that CD25 can be targeted for depletion of regulatory T cells in the therapeutic context, for example in an established solid tumour, and that CD25 is preferentially expressed in regulatory T cells. The present inventors have found that an engineered anti-CD25 antibody with enhanced binding to activatory Fc gamma receptors leads to effective depletion of tumour-infiltrating regulatory T cells, a therapeutic approach that could, for example, be associated (in combination with or within bispecific antibodies) with other cancer-targeting compounds, such as those targeting an immune checkpoint protein, a tumour-associated antigen, or an inhibitory Fcγ receptor.

The inventors have also found for the first time that inhibitory Fc gamma receptor IIb is upregulated at the tumour site, thus preventing effective intra-tumoural regulatory T cell depletion by the original anti-mouse CD25 antibody PC61. As such, described are therapeutic applications involving a combination approach involving targeting CD25 and Fc gamma receptor Ilb.

CD25 is the alpha chain of the IL-2 receptor, and is found on activated T cells, regulatory T cells, activated B cells, some thymocytes, myeloid precursors and oligodendrocytes. CD25 associates with CD122 and CD132 to form a heterotrimeric complex that acts as the high-affinity receptor for IL-2. The consensus sequence of human CD25 is shown below in SEQ ID NO:1 (Uniprot accession number P01589; the extracellular domain of mature human CD25, corresponding to amino acids 22-240, is underlined and is presented in SEQ ID NO:2):

10 20 30 40 50
MDSYLLMWGL LTFIMVPGCQ AELCDDDPPE IPHATFKAMA YKEGTMLNCE
60 70 80 90 100
CKRGFRRIKS GSLYMLCTGN SSHSSWDNQC QCTSSATRNT TKQVTPQPEE
110 120 130 140 150
QKERKTTEMQ SPMQPVDQAS LPGHCREPPP WENEATERIY HFVVGQMVYY
160 170 180 190 200
QCVQGYRALH RGPAESVCKM THGKTRWTQP QLICTGEMET SQFPGEEKPQ
210 220 230 240 250
ASPEGRPESE TSCLVTTTDF QIQTEMAATM ETSIFTTEYQ VAVAGCVFLL
260 270
ISVLLLSGLT WQRRQRKSRR TI

As used herein, "an antibody that binds CD25" refers to an antibody that is capable of binding to the CD25 subunit of the IL-2 receptor. This subunit is also known as the alpha subunit of the IL-2 receptor. Such an antibody is also referred to herein as an "anti-CD25 antibody".

An anti-CD25 antibody is an antibody capable of specific binding to the CD25 subunit (antigen) of the IL-2 receptor. "Specific binding", "bind specifically", and "specifically bind" are understood to mean that the antibody has a dissociation constant (K_{d}) for the antigen of interest of less than about 10⁻⁶ M, 10⁻⁷ M, 10⁻⁸ M, 10⁻⁹ M, 10⁻¹⁰ M, 10⁻¹¹ M or 10⁻¹² M. In a preferred embodiment the dissociation constant is less than 10⁻⁸ M, for instance in the range of 10⁻⁹ M, 10⁻¹⁰ M, 10⁻¹¹ M or 10⁻¹² M.

As used herein, the term "antibody" refers to intact immunoglobulin molecules and includes polyclonal, monoclonal, genetically engineered and otherwise modified forms of antibodies, including but not limited to chimeric antibodies, humanised antibodies, heteroconjugate and/or multispecific antibodies (e.g., bispecific antibodies, diabodies, tribodies, and tetrabodies). In some embodiments, an antibody may lack a covalent modification (e.g., attachment of a glycan) that it would have if produced naturally. In some embodiments, an antibody may contain a covalent modification (e.g., attachment of a glycan, a detectable moiety, a therapeutic moiety, a catalytic moiety, or other chemical group providing improved stability or administration of the antibody, such as poly-ethylene glycol). In some embodiments, an antibody is polyclonal or oligoclonal, that is generated as a panel of antibodies, each associated to a single antibody sequence and binding more or less distinct epitopes within an antigen (such as different epitopes within human CD25 extracellular domain that are associated to different reference anti-human CD25 antibodies). Polyclonal or oligoclonal antibodies can be provided in a single preparation for medical uses as described in the literature (Kearns JD et al., 2015. Mol Cancer Ther. 14:1625-36).

In one aspect of the invention the antibody is monoclonal. The antibody may additionally or alternatively be humanised or human. In a further aspect, the antibody is human, or in any case an antibody that has a format and features allowing its use and administration in human subjects.

Antibodies (Abs) and immunoglobulins (Igs) are glycoproteins having the same structural characteristics. Immunoglobulins may be from any class such as IgA, IgD, IgG, IgE or IgM. Immunoglobulins can be of any subclass such as IgG₁, IgG₂, IgG₃, or IgG₄. The anti-CD25 antibody may be from the IgG class, preferably the IgG₁ subclass. The anti-CD25 antibody is from the human IgG₁ subclass.

The Fc region of IgG antibodies interacts with several cellular Fcγ receptors (FcγR) to stimulate and regulate downstream effector mechanisms. There are five activating receptors, namely FcγRI (CD64), FcγRIIa (CD32a), FcγRIIc (CD32c), FcγRIIIa (CD16a) and FcγRIIIb (CD16b), and one inhibitory receptor FcγRIIb (CD32b). The communication of IgG antibodies with the immune system is controlled and mediated by FcγRs, which relay the information sensed and gathered by antibodies to the immune system, providing a link between the innate and adaptive immune systems, and particularly in the context of biotherapeutics (Hayes J et al., 2016. J Inflamm Res 9: 209-219).

IgG subclasses vary in their ability to bind to FcγR and this differential binding determines their ability to elicit a range of functional responses. For example, in humans, FcγRIIIa is the major receptor involved in the activation of antibody-dependent cell-mediated cytotoxicity (ADCC) and IgG3 followed closely by IgG1 display the highest affinities for this receptor, reflecting their ability to potently induce ADCC.

The antibody binds an activating FcγR receptor with high affinity. Preferably the antibody binds FcγRI and/or FcγRIIa and/or FcγRIIIa with high affinity. In a particular embodiment, the antibody binds to the FcγR with a dissociation constant of less than about 10⁻⁶ M, 10⁻⁷ M, 10⁻⁸ M, 10⁻⁹ M or 10⁻¹⁰ M.

The antibody is a human IgG₁ antibody, which is capable of binding to at least one Fc activating receptor. For example, the antibody may bind to one or more receptor selected from FcγRI, FcγRIIa, FcγRIIc, FcγRIIIa and FcγRIIIb. In one aspect the antibody is capable of binding to FcγRIIIa. In one aspect the antibody is capable of binding to FcγRIIIa and FcγRIIa and optionally FcγRI. In one aspect the antibody is capable of binding to these receptors with high affinity, for example with a dissociation constant of less than about 10⁻⁷ M, 10⁻⁸ M, 10⁻⁹ M or 10⁻¹⁰ M,

In one aspect the antibody binds an inhibitory receptor, FcγRIIb, with low affinity. In one aspect the antibody binds FcγRIIb with a dissociation constant higher than 10⁻⁷ M, higher than 10⁻⁶ M or higher than 10⁻⁵ M.

The anti-human CD25 antibody is from the human IgG₁ subclass, and preferably has ADCC and/or ADCP activity, as discussed herein, in particular with respect to cells of human origin. Indeed, As previously described (Nimmerjahn F et al., 2005. Science, 310:1510-2), the mlgG2a isotype (which corresponds human IgG1 isotype) binds to all FcγR subtypes with a high activatory to inhibitory ratio (A/I), that is at least superior to 1. In contrast, other isotypes (such as rIgG1 isotype) bind with a similar affinity to a single activatory FcγR only (FcγRIII), as well as the inhibitory FcγRIIb, resulting in a low A/I ratio (<1). As shown in the Examples, this lower A/I ratio correlates with a lower in intra-tumoral Treg depletion and lower anti-tumour therapeutic activity of the isotype.

In a preferred embodiment the anti-CD25 antibody as described herein binds human CD25, preferably with high affinity. Still preferably, the anti-CD25 antibody binds to extracellular region of human CD25, as shown above. In particular, the Examples provide experimental data generated with the antibody that is secreted by the PC-61.5.3 hybridoma and that generally identified as either PC61 or PC-61. The assays involving PC-61 and mouse CD25 in the literature (for example Setiady Y et al., 2010. Eur. J. Immunol. 40: 780-6; McNeill A et al., 2007. Scand J Immunol. 65:63-9; Teege S et al., 2015, Sci Rep 5: 8959), together with those disclosed in the Examples (including recombinant antibodies comprising CD25-binding domain of PC61), can be adapted for characterizing those human antibodies that recognize human CD25 having the same functional features of PC61 both at the level of interaction with CD25 (in particular, by blocking IL-2 binding) and with Fcγ receptors (in particular by preferably binding human activating Fcγ receptors and depleting efficiently Tregs), when the appropriate isotype is associated, as described in the Examples. Suitable methods will be known to one skilled in the art to achieve the required functional features of the antibody as described herein.

Described herein is a method of treating a human subject who has a cancer comprising the step of administering an anti-CD25 antibody to a subject, wherein said subject preferably has a solid tumour, and wherein the anti-CD25 antibody is preferably a human IgG1 antibody that binds to at least one activating Fcγ receptor selected from FcγRI (CD64), FcγRIIc (CD32c), and FcγRIIIa (CD16a) with high affinity, and depletes tumour-infiltrating regulatory T cells. Preferably the anti-CD25 antibody has a dissociation constant (K_{d}) for CD25 of less than 10⁻⁸ M. More preferably, the anti-CD25 antibody binds human CD25 providing effects on IL-2 binding and Treg depletion similar to those of on mouse CD25. In a further embodiment, the anti-CD25 antibody binds to Fcγ receptors with an activatory to inhibitory ratio (A/I) superior to 1 and/or binds to FcγRI (CD64), FcγRIIc (CD32c), FcγRIIIa (CD16a) with higher affinity than it binds to FcγRIIb (CD32b).

The CD25 binding domain of PC-61 antibody has been cloned and expressed as a recombinant protein in fusion with an appropriate constant region. The sequence of the CD25 binding domain of PC-61 antibody, as well its specificity for distinct epitopes within the extracellular domain of CD25 and/or its other functional activities, can be used for comparing candidate anti-CD25 antibodies that are generated and screened by any appropriate technique (e.g. by raising panels of hybridomas from CD25-immunized rodents or generating libraries of recombinant antibodies and then screening these antibody repertoires with CD25 fragments for characterizing functionally as described herein). The anti-CD25 antibodies that are consequently identified can be produced also as recombinant antibodies, in particular as full antibodies or as fragments or variants that are described herein.

Native antibodies and immunoglobulins are usually heterotetrameric glycoproteins of about 150,000 daltons, composed of two identical light (L) chains and two identical heavy (H) chains. Each heavy chain has at the amino terminus a variable domain (V_{H}) followed by a number of constant domains. Each light chain has a variable domain at the amino terminus (V_{L}) and a constant domain at the carboxy terminus.

The variable regions are capable of interacting with a structurally complementary antigenic target and are characterized by differences in amino acid sequence from antibodies of different antigenic specificity. The variable regions of either H or L chains contain the amino acid sequences capable of specifically binding to antigenic targets. Within these sequences are smaller sequences dubbed "hypervariable" because of their extreme variability between antibodies of differing specificity. Such hypervariable regions are also referred to as "complementarity determining regions" or "CDR" regions.

These CDR regions account for the basic specificity of the antibody for a particular antigenic determinant structure. The CDRs represent non-contiguous stretches of amino acids within the variable regions but, regardless of species, the positional locations of these critical amino acid sequences within the variable heavy and light chain regions have been found to have similar locations within the amino acid sequences of the variable chains. The variable heavy and light chains of all antibodies each have 3 CDR regions, each non-contiguous with the others (termed L1, L2, L3, H1, H2, H3) for the respective light (L) and heavy (H) chains. The accepted CDR regions have been described previously (Kabat et al., 1977. J Biol Chem 252, 6609-6616).

The antibodies of the present invention may function through complement-dependent cytotoxicity (CDC) and/or antibody-dependent cell-mediated cytotoxicity (ADCC) and/or antibody-dependent cell-mediated phagocytosis (ADCP), as well as any other mechanism that allows targeting, blocking proliferation, and/or depleting Treg cells.

"Complement-dependent cytotoxicity" (CDC) refers to lysis of antigen-expressing cells by an antibody of the invention in the presence of complement.

"Antibody-dependent cell-mediated cytotoxicity" (ADCC) refers to a cell-mediated reaction in which nonspecific cytotoxic cells that express Fc receptors (FcRs) (e.g. Natural Killer (NK) cells, neutrophils, and macrophages) recognize bound antibody on a target cell and thereby lead to lysis of the target cell.

"Antibody-dependent cell-mediated phagocytosis" (ADCP) refers to a cell-mediated reaction in which phagocytes (such as macrophages) that express Fc receptors (FcRs) recognize bound antibody on a target cell and thereby lead to phagocytosis of the target cell.

CDC, ADCC and ADCP can be measured using assays that are known and available in the art (Clynes et al. (1998) Proc Natl Acad Sci USA 95, 652-6). The constant region of an antibody is important in the ability of an antibody to fix complement and mediate cell-dependent cytotoxicity and phagocytosis. Thus, as discussed herein, the isotype of an antibody may be selected on the basis of whether it is desirable for the antibody to mediate cytotoxicity/phagocytosis.

As discussed herein, in an embodiment of the invention, an anti-CD25 antibody that leads to the depletion of Treg cells is used. For example, an anti-CD25 antibody that elicits a strong CDC response and/or a strong ADCC and/or a strong ADCP response may be used. Methods to increase CDC, ADCC and/or ADCP are known in the art. For example, CDC response may be increased with mutations in the antibody that increase the affinity of C1q binding (Idusogie et al. (2001) J Immunol 166, 2571-5).

ADCC may be increased by methods that eliminate the fucose moiety from the antibody glycan, such as by production of the antibody in a YB2/0 cell line, or though the introduction of specific mutations on the Fc portion of human IgG₁ (e.g., S298A/E333A/K334A, S239D/I332E/A330L, G236A/S239D/A330L/I332E) (Lazar et al. (2006) Proc Natl Acad Sci USA 103, 2005-2010*;* Smith et al. (2012) Proc Natl Acad Sci USA 109, 6181-6). ADCP may also be increased by the introduction of specific mutations on the Fc portion of human IgG1 (Richards et al. (2008) Mol Cancer Ther 7, 2517-27).

In a preferred embodiment of the present invention the antibody is optimised to elicit an ADCC response, that is to say the ADCC response is enhanced, increased or improved relative to other anti-CD25 antibodies, or example unmodified anti-CD25 monoclonal antibodies.

As used herein, a "chimeric antibody" can refer to an antibody having variable sequences derived from an immunoglobulin from one species, such as rat or mouse antibody, and immunoglobulin constant regions from another species, such as from a human antibody. In some embodiments, the chimeric antibody may have a constant region which is enhanced for inducing ADCC.

The antibodies according to the invention may also be partly or wholly synthetic, wherein at least part of the polypeptide chains of the antibodies are synthesized and, possibly, optimized for binding to their cognate antigen. Such antibodies may be chimeric or humanised antibodies and may be fully tetrameric in structure, or may be dimeric and comprise only a single heavy and a single light chain.

Antibodies of the present invention may also be monoclonal antibodies. As used herein, "monoclonal antibody" is not limited to antibodies produced through hybridoma technology. The term "monoclonal antibody" refers to an antibody that is derived from a single clone, including any eukaryotic, prokaryotic, or phage clone, and not the method by which it is produced.

The Antibodies described herein may also be human antibodies. As used herein, "human antibody" refers to antibodies having variable regions in which both the framework and CDR regions are derived from human germline immunoglobulin sequences. Furthermore, if the antibody contains a constant region, the constant region also is derived from human germline immunoglobulin sequences. The human antibodies may include amino acid residues not encoded by human germline immunoglobulin sequences (e.g., mutations introduced by random or site-specific mutagenesis *in vitro* or by somatic mutation *in vivo*)*.*

Also described are nucleic acid molecules encoding anti-CD25 antibodies as defined herein. Such provided nucleic acid molecules may contain codon-optimized nucleic acid sequences, and/or may be included in expression cassettes within appropriate nucleic acid vectors for the expression in host cells such as, for example, bacterial, yeast, insect, piscine, murine, simian, or human cells. Also described are host cells comprising heterologous nucleic acid molecules (e.g. DNA vectors) that express the desired antibody.

Also described are methods of preparing an isolated anti-CD25 antibody as defined above. Such methods may comprise culturing a host cell that comprises nucleic acids (e.g., heterologous nucleic acids that may comprise and/or be delivered to the host cell via vectors). Preferably, the host cell (and/or the heterologous nucleic acid sequences) is/are arranged and constructed so that the antibody or antigen-binding fragment thereof is secreted from the host cell and isolated from cell culture supernatants

The antibodies of the present invention may be monospecific, bispecific, or multispecific. "Multispecific antibodies" may be specific for different epitopes of one target antigen or polypeptide, or may contain antigen-binding domains specific for more than one target antigen or polypeptide (Kufer et al. (2004) Trends Biotechnol 22, 238-44).

In one aspect of the invention the antibody is a monospecific antibody. As discussed further below, in an alternative aspect the antibody is a bispecific antibody.

As used herein, "bispecific antibody" refers to an antibody having the capacity to bind to two distinct epitopes either on a single antigen or polypeptide, or on two different antigens or polypeptides.

Bispecific antibodies as discussed herein can be produced via biological methods, such as somatic hybridization; or genetic methods, such as the expression of a non-native DNA sequence encoding the desired antibody structure in cell line or in an organism; chemical methods (e.g., by chemical coupling, genetic fusion, noncovalent association or otherwise to one or more molecular entities such as another antibody or antibody fragment); or a combination thereof.

The technologies and products that allow producing monospecific or bispecific are known in the art, as extensively reviewed in the literature, also with respect to alternative formats, antibody-drug conjugates, antibody design methods, in vitro screening methods, constant regions, post-translational and chemical modifications, improved feature for triggering cancer cell death such as Fc engineering (Tiller K and Tessier P, 2015 Annu Rev Biomed Eng. 17: 191-216; Speiss C et al., 2015. Molecular Immunology 67: 95-106; Weiner G, 2015. Nat Rev Cancer, 15: 361-370; Fan G et al., 2015. J Hematol Oncol 8:130).

As used herein, "epitope" or "antigenic determinant" refers to a site on an antigen to which an antibody binds. As is well known in the art, epitopes can be formed both from contiguous amino acids (linear epitope) or non-contiguous amino acids juxtaposed by tertiary folding of a protein (conformational epitopes). Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes are well known in the art and include, for example, x-ray crystallography and 2-D nuclear magnetic resonance. See, for example, Epitope Mapping Protocols in Methods in Molecular Biology, Vol. 66, Glenn E. Morris, Ed (1996).

The anti-CD25 antibody can be included in an agent that further comprises a conjugated payload such as a therapeutic or diagnostic agent, in particular for cancer therapy or diagnosis. Anti-CD25 antibody conjugates with radionuclides or toxins may be used. Examples of commonly used radionuclides are, for example, ⁹⁰Y, ¹³¹I, and ⁶⁷Cu, among others, and examples of commonly used toxins are doxorubicin and calicheamicin. In a further embodiment, the anti-CD25 antibody may be modified to have an altered half-life. Methods for achieving an altered half life are known in the art.

In one embodiment the antibody may block the function of human CD25, preferably in addition to promoting depletion (through ADCC, ADCP and/or CDC) of CD25-expressing cells. Preferably it also blocks the binding of human IL-2 to human CD25, and most preferably blocks human IL-2 signalling in CD25-expressing cells.

As described herein the subject of the treatment can be a mammal, preferably a cat, dog, horse, donkey, sheep, pig, goat, cow, hamster, mouse, rat, rabbit or guinea pig, but most preferably the subject is a human. In all aspects of the invention the subject is a human.

As used herein, the terms "cancer", "cancerous", or "malignant" refer to or describe the physiological condition in mammals that is typically characterized by unregulated cell growth.

Examples of cancer include but are not limited to, carcinoma, lymphoma, leukemia, blastoma, and sarcoma. More particular examples of such cancers include squamous cell carcinoma, myeloma, small-cell lung cancer, non-small cell lung cancer, glioma, hepatocellular carcinoma (HCC), hodgkin's lymphoma, non- hodgkin's lymphoma, acute myeloid leukemia (AML), multiple myeloma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, stomach cancer, bladder cancer, hepatoma, breast cancer, colon carcinoma, and head and neck cancer.

In one aspect the cancer involves a solid tumour. Examples of solid tumours are sarcomas (including cancers arising from transformed cells of mesenchymal origin in tissues such as cancellous bone, cartilage, fat, muscle, vascular, hematopoietic, or fibrous connective tissues), carcinomas (including tumors arising from epithelial cells), mesothelioma, neuroblastoma, retinoblastoma, etc. Cancers involving solid tumours include, without limitations, brain cancer, lung cancer, stomach cancer, duodenal cancer, esophagus cancer, breast cancer, colon and rectal cancer, renal cancer, bladder cancer, kidney cancer, pancreatic cancer, prostate cancer, ovarian cancer, melanoma, mouth cancer, sarcoma, eye cancer, thyroid cancer, urethral cancer, vaginal cancer, neck cancer, lymphoma, and the like.

In a one aspect of the invention the cancer is selected from melanoma, non-small cell lung cancer, renal cancer, ovarian cancer, bladder cancer, sarcoma and colon cancer. In a preferred aspect of the invention the cancer is selected from melanoma, ovarian, non-small cell lung cancer and renal cancer. In one embodiment the cancer is not melanoma, ovarian cancer, or breast cancer. In a preferred aspect, the cancer is sarcoma, colon, melanoma or colorectal cancer, or more generally any human cancer for which the MCA205, CT26, B16, or MC38 cell line (as identified in the Examples) may represent preclinical models for validating compounds as being useful for their therapeutic management.

As used herein, the term "tumour" as it applies to a subject diagnosed with, or suspected of having, a cancer refers to a malignant or potentially malignant neoplasm or tissue mass of any size, and includes primary tumours and secondary neoplasms. The terms "cancer", "malignancy", "neoplasm", "tumor", and "carcinoma can be also used interchangeably herein to refer to tumours and tumour cells that exhibit relatively abnormal, uncontrolled, and/or autonomous growth, so that they exhibit an aberrant growth phenotype characterized by a significant loss of control of cell proliferation. In general, cells of interest for detection or treatment include precancerous (e.g., benign), malignant, pre-metastatic, metastatic, and non-metastatic cells. The teachings of the present disclosure may be relevant to any and all cancers.

As used herein, "solid tumours" are an abnormal growth or mass of tissue that usually does not contain cysts or liquid areas, in particular, tumours and/or metastasis (wherever located) other than leukaemia or non-solid lymphatic cancers. Solid tumours may be benign or malignant. Different types of solid tumours are named for the type of cells that form them and/or the tissue or organ in which they are located. Examples of solid tumours are sarcomas (including cancers arising from transformed cells of mesenchymal origin in tissues such as cancellous bone, cartilage, fat, muscle, vascular, hematopoietic, or fibrous connective tissues), carcinomas (including tumours arising from epithelial cells), melanomas, lymphomas, mesothelioma, neuroblastoma, and retinoblastoma.

Cancers in accordance with the present invention include those characterized by the presence of a solid tumour, that is to say the subject does not have a non-solid tumour. In all aspects of the invention as discussed herein the cancer is a solid tumour, i.e. that the subject has a solid tumour (and does not have a non-solid tumour).

Reference to "treat" or "treating" a cancer as used herein defines the achievement of at least one positive therapeutic effect, such as for example, reduced number of cancer cells, reduced tumour size, reduced rate of cancer cell infiltration into peripheral organs, or reduced rate of tumour metastasis or tumour growth.

Positive therapeutic effects in cancer can be measured in a number of ways (e.g. Weber (2009) J Nucl Med 50, 1S-10S*).* By way of example, with respect to tumour growth inhibition, according to National Cancer Institute (NCI) standards, a T/C ≤ 42% is the minimum level of anti-tumour activity. A T/C < 10% is considered a high anti-tumour activity level, with T/C (%) = Median tumour volume of the treated/Median tumour volume of the control x 100. In some embodiments, the treatment achieved by a therapeutically effective amount is any of progression free survival (PFS), disease free survival (DFS) or overall survival (OS). PFS, also referred to as "Time to Tumour Progression" indicates the length of time during and after treatment that the cancer does not grow, and includes the amount of time patients have experienced a complete response or a partial response, as well as the amount of time patients have experienced stable disease. DFS refers to the length of time during and after treatment that the patient remains free of disease. OS refers to a prolongation in life expectancy as compared to naive or untreated individuals or patients.

Reference to "prevention" (or prophylaxis) as used herein refers to delaying or preventing the onset of the symptoms of the cancer. Prevention may be absolute (such that no disease occurs) or may be effective only in some individuals or for a limited amount of time.

In a preferred aspect of the invention the subject has an established tumour, that is the subject already has a tumour, e.g. that is classified as a solid tumour. As such, the invention as described herein can be used when the subject already has a tumour, such as a solid tumour. As such, the invention provides a therapeutic option that can be used to treat an existing tumour. In one aspect of the invention the subject has an existing solid tumour. The invention may be used as a prevention, or preferably as a treatment in subjects who already have a solid tumour. In one aspect the invention is not used as a preventative or prophylaxis.

In one aspect tumour regression may be enhanced, tumour growth may be impaired or reduced, and/or survival time may be enhanced using the invention as described herein, for example compared with other cancer treatments (for example standard-of care treatments for the a given cancer).

The method of treating or preventing cancer as described herein further comprises the step of identifying a subject who has cancer, in particular identifying a subject who has a tumour such as a solid tumour.

The dosage regimen of a therapy described herein that is effective to treat a cancer patient may vary according to factors such as the disease state, age, and weight of the patient, and the ability of the therapy to elicit an anti-cancer response in the subject. Selection of an appropriate dosage will be within the capability of one skilled in the art. For example 0.01, 0.1, 0.3, 0.5, 1, 2, 3, 4, 5, 6, 7, 8, 9, 10, 20, 30, 40, or 50 mg/kg. In some embodiments, such quantity is a unit dosage amount (or a whole fraction thereof) appropriate for administration in accordance with a dosing regimen that has been determined to correlate with a desired or beneficial outcome when administered to a relevant population (i.e., with a therapeutic dosing regimen).

The antibody according to any aspect of the invention as described herein may be in the form of a pharmaceutical composition which additionally comprises a pharmaceutically acceptable carrier, diluent or excipient. These compositions include, for example, liquid, semi-solid and solid dosage formulations, such as liquid solutions (e.g., injectable and infusible solutions), dispersions or suspensions, tablets, pills, or liposomes. In some embodiments, a preferred form may depend on the intended mode of administration and/or therapeutic application. Pharmaceutical compositions containing the antibody can be administered by any appropriate method known in the art, including, without limitation, oral, mucosal, by-inhalation, topical, buccal, nasal, rectal, or parenteral (e.g. intravenous, infusion, intratumoural, intranodal, subcutaneous, intraperitoneal, intramuscular, intradermal, transdermal, or other kinds of administration involving physical breaching of a tissue of a subject and administration of the pharmaceutical composition through the breach in the tissue). Such a formulation may, for example, be in a form of an injectable or infusible solution that is suitable for intradermal, intratumoural or subcutaneous administration, or for intravenous infusion. The administration may involve intermittent dosing. Alternatively, administration may involve continuous dosing (e.g., perfusion) for at least a selected period of time, simultaneously or between the administration of other compounds.

In some embodiments, the antibody can be prepared with carriers that protect it against rapid release and/or degradation, such as a controlled release formulation, such as implants, transdermal patches, and microencapsulated delivery systems. Biodegradable, biocompatible polymers can be used.

Those skilled in the art will appreciate, for example, that route of delivery (e.g., oral vs intravenous vs subcutaneous vs intratumoural, etc) may impact dose amount and/or required dose amount may impact route of delivery. For example, where particularly high concentrations of an agent within a particular site or location (e.g., within a tumour) are of interest, focused delivery (e.g., in this example, intratumoural delivery) may be desired and/or useful. Other factors to be considered when optimizing routes and/or dosing schedule for a given therapeutic regimen may include, for example, the particular cancer being treated (e.g., type, stage, location, etc.), the clinical condition of a subject (e.g., age, overall health, etc.), the presence or absence of combination therapy, and other factors known to medical practitioners.

The pharmaceutical compositions typically should be sterile and stable under the conditions of manufacture and storage. The composition can be formulated as a solution, microemulsion, dispersion, liposome, or other ordered structure suitable to high drug concentration. Sterile injectable solutions can be prepared by incorporating the antibody in the required amount in an appropriate solvent with one or a combination of ingredients enumerated above, as required, followed by filtered sterilization.. Formulations for parenteral administration include, but are not limited to, suspensions, solutions, emulsions in oily or aqueous vehicles, pastes, and implantable sustained-release or biodegradable formulations as discussed herein. Sterile injectable formulations may be prepared using a non-toxic parenterally acceptable diluent or solvent. Each pharmaceutical composition for use in accordance with the present invention may include pharmaceutically acceptable dispersing agents, wetting agents, suspending agents, isotonic agents, coatings, antibacterial and antifungal agents, carriers, excipients, salts, or stabilizers are non-toxic to the subjects at the dosages and concentrations employed. Preferably, such a composition can further comprise a pharmaceutically acceptable carrier or excipient for use in the treatment of cancer that that is compatible with a given method and/or site of administration, for instance for parenteral (e.g. sub-cutaneous, intradermal, or intravenous injection), intratumoral, or peritumoral administration.

While an embodiment of the compositions for use according to the present invention may not be effective in achieving a positive therapeutic effect in every subject, it should do so in a using pharmaceutical compositions and dosing regimens that are consistently with good medical practice and statistically significant number of subjects as determined by any statistical test known in the art such as the Student's t-test, the X²-test, the U-test according to Mann and Whitney, the Kruskal-Wallis test (H-test), Jonckheere-Terpstra test and the Wilcoxon-test.

Where hereinbefore and subsequently a tumour, a tumour disease, a carcinoma or a cancer is mentioned, also metastasis in the original organ or tissue and/or in any other location are implied alternatively or in addition, whatever the location of the tumour and/or metastasis is.

As discussed herein, the present invention relates to depleting regulatory T cells (Tregs). Thus, in one aspect of the invention, the anti-CD25 antibody depletes or reduces tumour-infiltrating regulatory T cells. In one aspect said depletion is via ADCC. In another aspect, said depletion is via ADCP. The anti-CD25 antibody may also deplete or reduce circulating regulatory T cells. In one aspect said depletion is via ADCC. In another aspect, said depletion is via ADCP.

As such, also described is a method for depleting regulatory T cells in a tumour in a subject, comprising administering to said subject an anti-CD25 antibody. In a preferred embodiment Tregs are depleted in a solid tumour. By "depleted" it is meant that the number, ratio or percentage of Tregs is decreased relative to when an anti-CD25 antibody is not administered. In particular embodiments of the invention as described herein, over about 5%, 10%, 20%, 30%, 40%, 50%, 60%, 70%, 80%, 90% or 99% of the tumour-infiltrating regulatory T cells are depleted.

As used herein, "regulatory T cells" ("Treg", "Treg cells", or "Tregs") refer to a lineage of CD4+ T lymphocytes specialized in controlling autoimmunity, allergy and infection. Typically, they regulate the activities of T cell populations, but they can also influence certain innate immune system cell types. Tregs are usually identified by the expression of the biomarkers CD4, CD25 and Foxp3. Naturally occurring Treg cells normally constitute about 5-10% of the peripheral CD4+ T lymphocytes. However, within a tumour microenvironment (i.e. tumour-infiltrating Treg cells), they can make up as much as 20-30% of the total CD4+ T lymphocyte population.

Activated human Treg cells may directly kill target cells such as effector T cells and APCs through perforin- or granzyme B-dependent pathways; cytotoxic T-lymphocyte-associated antigen 4 (CTLA4+) Treg cells induce indoleamine 2,3-dioxygenase (IDO) expression by APCs, and these in turn suppress T-cell activation by reducing tryptophan; Treg cells, may release interleukin-10 (IL-10) and transforming growth factor (TGFβ) *in vivo,* and thus directly inhibit T-cell activation and suppress APC function by inhibiting expression of MHC molecules, CD80, CD86 and IL-12. Treg cells can also suppress immunity by expressing high levels of CTLA4 which can bind to CD80 and CD86 on antigen presenting cells and prevent proper activation of effector T cells.

In a preferred embodiment of the present invention the ratio of effector T cells to regulatory T cells in a solid tumour is increased. In some embodiments, the ratio of effector T cells to regulatory T cells in a solid tumour is increased to over 5, 10, 15, 20, 40 or 80.

An immune effector cell refers to an immune cell which is involved in the effector phase of an immune response. Exemplary immune cells include a cell of a myeloid or lymphoid origin, e.g., lymphocytes (e.g., B cells and T cells including cytolytic T cells (CTLs)), killer cells, natural killer cells, macrophages, monocytes, eosinophils, neutrophils, polymorphonuclear cells, granulocytes, mast cells, and basophils.

Immune effector cells involved in the effector phase of an immune response express specific Fc receptors and carry out specific immune functions. An effector cell can induce antibody-dependent cell-mediated cytotoxicity (ADCC), e.g., a neutrophil capable of inducing ADCC. For example, monocytes, macrophages, neutrophils, eosinophils, and lymphocytes which express FcαR are involved in specific killing of target cells and presenting antigens to other components of the immune system, or binding to cells that present antigens. An effector cell can also phagocytose a target antigen, target cell, or microorganism. As discussed herein, antibodies according to the present invention may be optimised for ability to induce ADCC.

In some embodiments, a different agent against cancer may be administered in combination with the antibody via the same or different routes of delivery and/or according to different schedules. Alternatively or additionally, in some embodiments, one or more doses of a first active agent is administered substantially simultaneously with, and in some embodiments via a common route and/or as part of a single composition with, one or more other active agents. Those skilled in the art will further appreciate that some embodiments of combination therapies provided in accordance with the present invention achieve synergistic effects; in some such embodiments, dose of one or more agents utilized in the combination may be materially different (e.g., lower) and/or may be delivered by an alternative route, than is standard, preferred, or necessary when that agent is utilized in a different therapeutic regimen (e.g., as monotherapy and/or as part of a different combination therapy).

In some embodiments, where two or more active agents are utilized in accordance with the present invention, such agents can be administered simultaneously or sequentially. In some embodiments, administration of one agent is specifically timed relative to administration of another agent. For example, in some embodiments, a first agent is administered so that a particular effect is observed (or expected to be observed, for example based on population studies showing a correlation between a given dosing regimen and the particular effect of interest). In some embodiments, desired relative dosing regimens for agents administered in combination may be assessed or determined empirically, for example using ex vivo, in vivo and/or in vitro models; in some embodiments, such assessment or empirical determination is made in vivo, in a patient population (e.g., so that a correlation is established), or alternatively in a particular patient of interest.

The present inventors have shown that an anti-CD25 antibody shows improved therapeutic effects when combined with an immune checkpoint inhibitor. As shown in the present Examples, a combination therapy with an anti-CD25 antibody and an immune checkpoint inhibitor can have synergistic effects in the treatment of established tumours. The data in respect of PD-1/PD-L1 in the present Examples relates to interfering with PD-1/PD-L1 interaction. As such, the interaction between the PD-1 receptor and the PD-L1 ligand may be blocked, resulting in "PD-1 blockade". In one aspect the combination may lead to enhanced tumour regression, enhanced impairment or reduction of tumour growth, and/or survival time may be enhanced using the invention as described herein, for example compared with either anti-CD25 antibodies or PD-1/PD-L1 blockade alone (directly, using an anti-PD1 antibody, or indirectly, using an anti-PD-L1 antibody).

As used herein, "immune checkpoint" or "immune checkpoint protein" refer to proteins belonging to inhibitory pathways in the immune system, in particular for the modulation of T-cell responses. Under normal physiological conditions, immune checkpoints are crucial to preventing autoimmunity, especially during a response to a pathogen. Cancer cells are able to alter the regulation of the expression of immune checkpoint proteins in order to avoid immune surveillance.

Examples of immune checkpoint proteins include but are not limited to PD-1, CTLA-4, BTLA, KIR, LAG3, TIGIT, CD155, B7H3, B7H4, VISTA and TIM3, and also OX40, GITR, ICOS, 4-1BB and HVEM. Immune checkpoint proteins may also refer to proteins which bind to other immune checkpoint proteins which modulate the immune response in an inhibitory manner. Such proteins include PD-L1, PD-L2, CD80, CD86, HVEM, LLT1, and GAL9.

"Immune checkpoint protein inhibitors" refer to any protein that can interfere with the signalling and/or protein-protein interactions mediated by an immune checkpoint protein. In one aspect of the invention the immune checkpoint protein is PD-1 or PD-L1. In a preferred aspect of the invention as described herein the immune checkpoint inhibitor interferes with PD-1/PD-L1 interactions via anti-PD-1 or anti PD-L1 antibodies.

As such, also described is a method of treating cancer, comprising administering an anti-CD25 antibody and a checkpoint inhibitor to a subject. The invention also provides an anti-CD25 antibody and an immune checkpoint inhibitor for use in the treatment of cancer.

Also described is the use of an anti-CD25 antibody and an immune checkpoint inhibitor for the manufacture of a medicament for the treatment of cancer. Administration of the anti-CD25 antibody and immune checkpoint inhibitor may be simultaneous, separate or sequential.

The present invention provides a combination of an anti-CD25 antibody and an immune checkpoint inhibitor for use in the treatment of cancer in a subject, wherein the anti-CD25 antibody and the immune checkpoint inhibitor are administered simultaneously, separately or sequentially. Such an anti-human CD25 antibody is a human IgG1 and can be used specifically in combination with antibodies targeting immune checkpoints that either present or lack sequences that allow ADCC, ADCP, and/or CDC.

Also described is an anti-CD25 antibody for use in the treatment of cancer, wherein said antibody is for administration in combination with an immune checkpoint inhibitor. Also described is the use of an anti-CD25 antibody in the manufacture of a medicament for treating cancer, wherein said medicament is for administration in combination with an immune checkpoint inhibitor.

Also described is a pharmaceutical composition comprising an anti- CD25 antibody and an immune checkpoint inhibitor in a pharmaceutically acceptable medium. As discussed above, the immune checkpoint inhibitor may be an inhibitor of PD-1, i.e. a PD-1 antagonist.

PD-1 (Programmed cell Death protein 1), also known as CD279, is a cell surface receptor expressed on activated T cells and B cells. Interaction with its ligands has been shown to attenuate T-cell responses both *in vitro* and *in vivo.* PD-1 binds two ligands, PD-L1 and PD-L2. PD-1 belongs to the immunoglobulin superfamily. PD-1 signalling requires binding to a PD-1 ligand in close proximity to a peptide antigen presented by major histocompatibility complex (MHC) (Freeman (2008) Proc Natl Acad Sci USA 105, 10275-6). Therefore, proteins, antibodies or small molecules that prevent co-ligation of PD-1 and TCR on the T cell membrane are useful PD-1 antagonists.

In one embodiment, the PD-1 receptor antagonist is an anti-PD-1 antibody, or an antigen binding fragment thereof, which specifically binds to PD-1 and blocks the binding of PD-L1 to PD-1. The anti-PD-1 antibody may be a monoclonal antibody. The anti-PD-1 antibody may be a human or humanised antibody. An anti-PD-1 antibody is an antibody capable of specific binding to the PD-1 receptor. Anti-PD-1 antibodies known in the art include Nivolumab and Pembrolizumab.

PD-1 antagonists also include compounds or agents that either bind to and/or block a ligand of PD-1 to interfere with or inhibit the binding of the ligand to the PD-1 receptor, or bind directly to and block the PD-1 receptor without inducing inhibitory signal transduction through the PD-1 receptor. Alternatively, the PD-1 receptor antagonist can bind directly to the PD-1 receptor without triggering inhibitory signal transduction and also binds to a ligand of the PD-1 receptor to reduce or inhibit the ligand from triggering signal transduction through the PD-1 receptor. By reducing the number and/or amount of ligands that bind to PD-1 receptor and trigger the transduction of an inhibitory signal, fewer cells are attenuated by the negative signal delivered by PD-1 signal transduction and a more robust immune response can be achieved.

In one embodiment, the PD-1 receptor antagonist is an anti-PD-L1 antibody, or an antigen binding fragment thereof, which specifically binds to PD-L1 and blocks the binding of PD-L1 to PD-1. The anti-PD-L1 antibody may be a monoclonal antibody. The anti-PD-L1 antibody may be a human or humanised antibody, such as Atezolizumab (MPDL3280A).

Also described is a method of treating cancer, comprising administering an anti-CD25 antibody and an antibody which is an agonist of a T cell activating costimulatory pathway to a subject. Antibody agonists of a T cell activating costimulatory pathway include, without limitation, agonist antibodies against ICOS, GITR, OX40, CD40, LIGHT and 4-1BB.

The present inventors have identified that, surprisingly, the level of the inhibitory Fc receptor, FcγRIIb (CD32b), may be increased in solid tumours. Thus, a further method of treating cancer comprises administering an anti-CD25 antibody and a compound that decreases, blocks, inhibits, and/or antagonizes FcγRIIb (CD32b). Such FcγRIIb antagonist can be a small molecule interfering for FcγRIIb-induced intracellular signalling, modified antibodies that do not engage inhibitory FcγRIIb receptor, or an anti-human FcγRIIb (anti-CD32b antibody. For example, antagonistic anti-human FcγRIIb antibodies have been characterized also for their anti-tumour properties (Roghanian A et al., 2015, Cancer Cell. 27, 473-488; Rozan C et al., 2013, Mol Cancer Ther. 12:1481-91; WO2015173384; WO2008002933).

Also described is a bispecific antibody comprising:
(a) a first antigen binding moiety that binds to CD25; and
(b) a second antigen binding moiety that binds to an immune checkpoint protein, a tumour-associated antigen, is (or is based on) an anti-human activatory Fc Receptor antibody (for example anti-FcgRI, anti-FcgRIIa, anti-FcgRIII), or is (or is based on) an antagonistic anti-human FcγRIIb antibody;
wherein the bispecific antibody is is preferrably an IgG1 antibody that binds to at least one activatory Fcγ receptor with high affinity, and depletes tumour-infiltrating regulatory T cells.

As used herein, "tumour-associated antigen" refers to antigens expressed on tumour cells, making them distinguishable from non-cancer cells adjacent to them, and include, without limitation, CD20, CD38, PD-L1, EGFR, EGFRV3, CEA, TYRP1 and HER2. Various review articles have been published that describe relevant tumour-associated antigens and the corresponding therapeutically useful antitumor antibody agents (see, for example, Sliwkowski & Mellman (2013) Science 341, 192-8). Such antigens and corresponding antibodies include, without limitation CD22 (Blinatumomab), CD20 (Rituximab, Tositumomab), CD56 (Lorvotuzumab), CD66e/CEA (Labetuzumab), CD152/CTLA-4 (Ipilimumab), CD221/IGF1R (MK-0646), CD326/Epcam (Edrecolomab), CD340/HER2 (Trastuzumab, Pertuzumab), and EGFR (Cetuximab, Panitumumab).

In one aspect, the bispecific antibody as described herein leads to ADCC, or, in one aspect, enhanced ADCC.

The bispecific antibody may bind to a specific epitope on CD25, and a specific epitope on the immune checkpoint protein or tumour-associated antigen as defined herein. The second antigen binding moiety may bind to PD-L1. Also described is a bispecific antibody comprising:
(a) a first antigen binding moiety that binds to CD25; and
(b) a second antigen binding moiety that binds to an immune checkpoint protein expressed on a tumour cell.

The immune checkpoint protein expressed on a tumour cell may be PD-L1, VISTA, GAL9, B7H3 or B7H4. Still preferably, the anti-CD25 antibody is an IgG1 antibody that binds to the Fcγ receptors with high affinity, and depletes tumour-infiltrating regulatory T cells.

One skilled in the art would be able to produce a bispecific antibody using known methods. The bispecific antibody described herein may be used in any of the aspects as described herein. Preferably, the second antigen binding moiety within the bispecific antibody according to the invention binds to human PD-1, human PD-L1, or human CTLA-4.

In one aspect the bispecific antibody may bind to CD25 and to immune modulatory receptors expressed at high levels on tumour infiltrating Tregs, for example CTLA4, ICOS, GITR, 4-1BB or OX40.

Also described is a kit which comprises an anti-CD25 antibody as described herein, and an immune checkpoint inhibitor, preferably a PD-1 antagonist (directly, using an anti-PD1 antibody, or indirectly, using an anti-PD-L1 antibody) as discussed herein. In one aspect the immune checkpoint inhibitor is anti-PD-L1. Alternatively the kit may comprise an anti-CD25 antibody as described herein, and an antibody which is an agonist of a T cell activating costimulatory pathway. The kit may comprise instructions for use.

In a further aspect the kit may comprise an anti-CD25 antibody as described herein and a compound that decreases, blocks, inhibits, and/or antagonizes FcγRIIb (CD32b), or alternatively an anti-CD25 antibody as described herein and an anti-human activatory Fc Receptor antibody (anti-FcγRI, anti-FcγRIIc, or anti-FcγRIIIa).

Any aspect as described herein may be performed in combination with additional cancer therapies. In particular, the anti-CD25 antibody and immune checkpoint inhibitor (or any other combination therapy) may be administered in combination with co-stimulatory antibodies, chemotherapy and/or radiotherapy, targeted therapy or monoclonal antibody therapy.

A chemotherapeutic entity as used herein refers to an entity which is destructive to a cell, that is the entity reduces the viability of the cell. The chemotherapeutic entity may be a cytotoxic drug. A chemotherapeutic agent contemplated includes, without limitation, alkylating agents, anthracyclines, epothilones, nitrosoureas, ethylenimines/methylmelamine, alkyl sulfonates, alkylating agents, antimetabolites, pyrimidine analogs, epipodophylotoxins, enzymes such as L-asparaginase; biological response modifiers such as IFNα, IFN-γ, IL-2, IL-12, G-CSF and GM-CSF; platinum coordination complexes such as cisplatin, oxaliplatin and carboplatin, anthracenediones, substituted urea such as hydroxyurea, methylhydrazine derivatives including N-methylhydrazine (MIH) and procarbazine, adrenocortical suppressants such as mitotane (o,p'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide.

The additional cancer therapy may also include the administration of a cancer vaccine. "Cancer vaccines" as used herein refer to therapeutic cancer vaccines administrated to cancer patients and designed to eradicate cancer cells through strengthening patient's own immune responses. Cancer vaccines include tumour cell vaccines (autologous and allogenic), dendritic cell vaccines (*ex vivo* generated and peptide-activated), protein/peptide-based cancer vaccines and genetic vaccines (DNA, RNA and viral based vaccines). Accordingly, therapeutic cancer vaccines, in principle, may be utilized to inhibit further growth of advanced cancers and/or relapsed tumours that are refractory to conventional therapies, such as surgery, radiation therapy and chemotherapy. Tumour cell based vaccines (autologous and allogeneic) include those genetically modified to secrete soluble immune stimulatory agents such as cytokines (IL2, IFN-g, IL12, GMCSF, FLT3L), single chain Fv antibodies against immune modulatory receptors (PD-1, CTLA-4, GITR, ICOS, OX40, 4-1BB) and/or to express on their membrane the ligand for immune-stimulatory receptors such as ICOS-ligand, 4-1BB ligand, GITR-ligand, and/or OX40 ligand amongst others.

The additional cancer therapy may be other antibodies or small molecule reagents that reduce immune regulation in the periphery and within the tumour microenvironment, for example molecules that target TGFb pathways, IDO (indoleamine deoxigenase), Arginase, and/or CSF1R.

'In combination' may refer to administration of the additional therapy before, at the same time as or after administration of any aspect according to the present invention.

The invention will now be further described by way of the following Examples, which are meant to serve to assist one of ordinary skill in the art in carrying out the invention with reference to the drawings in which:

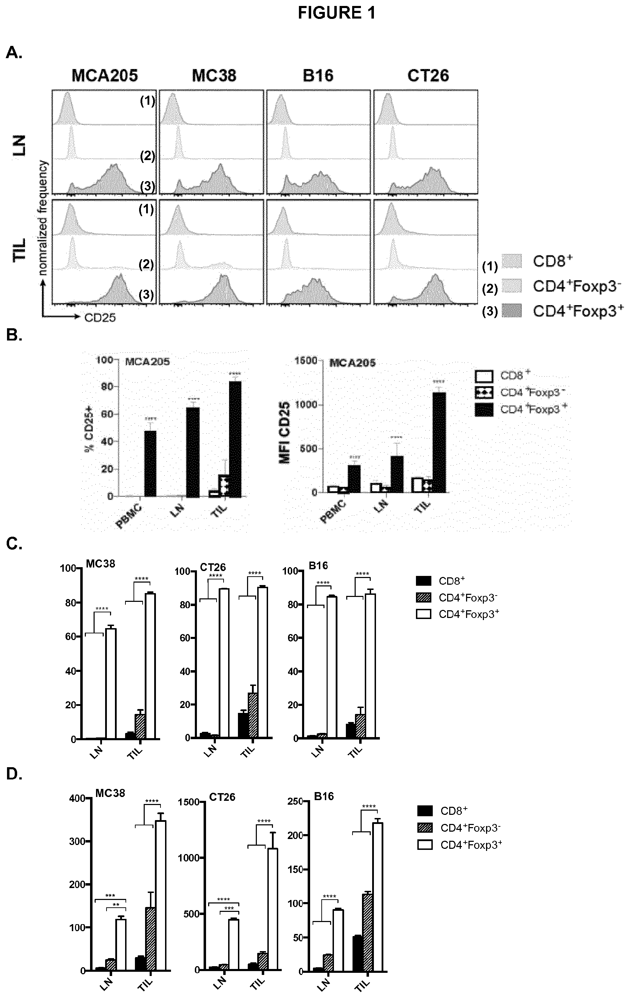

**Figure 1** - shows the expression of CD25pos regulatory T cells to blood and lymph node (A) Expression of CD25 (detection antibody clone 7D4; anti-mouse CD25, IgM isotype) on the surface of T cell subsets that are present in lymph nodes (LN) and tumour infiltrating lymphocytes (TIL) of different tumour models. Histograms are representative of one mouse for each tumour model. (B) Percentage of CD25 positive cells and MFI of CD25 in PBMC and T cell subsets from pooled data (n=10) of individual experiments using the MCA205 tumour model. The same evaluation (restricted to T cell subsets) have been performed in MC38, B16, and CT26 tumour models in (C) and (D). Error bars represent standard error (SE) of the mean. Statistical relevance between CD4-positive, Foxp3-positive cells and CD8-positive or CD4-positive / Foxp3-negative cells is indicated.

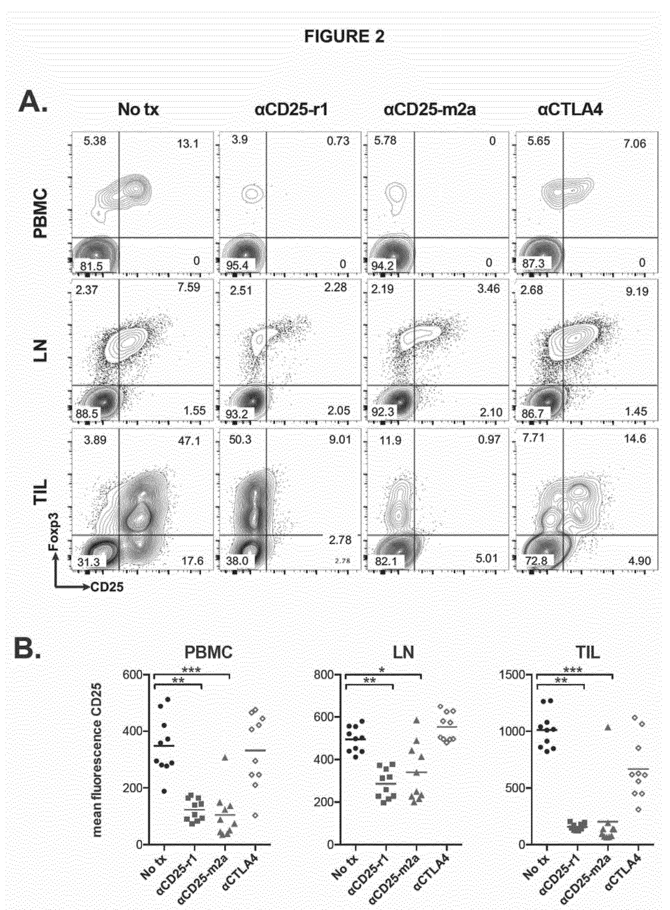

**Figure 2** - shows the restriction of anti-CD25 (αCD25)-mediated depletion of CD25-positive, regulatory T cells to blood and lymph node in MCA205 tumour model. (A) Expression of CD25 (detection antibody clone 7D4; anti- mouse CD25, IgM isotype) and FoxP3 in CD4-positive T cells. (B) Mean fluorescence intensity of CD25 on Treg (gated on CD4-positive, FoxP3-positive T cells). Tumor-bearing mice were injected with 200 µg of anti-CD25-r1 (αCD25-r1; anti-CD25 rat IgG1), anti-CD25-m2a (αCD25-m2a; anti-CD25 murine IgG2a), anti-CTLA-4 (αCTLA-4; anti-CTLA4 clone B56), or not treated (no tx) on days 5 and 7 after s.c. inoculation with 5 x 10⁵ MCA205 cells. Peripheral blood mononuclear cells (PBMC), lymph nodes (LN) and tumor infiltrating lymhocytes (TIL) were harvested on day 9, processed and stained for flow cytometry analysis.

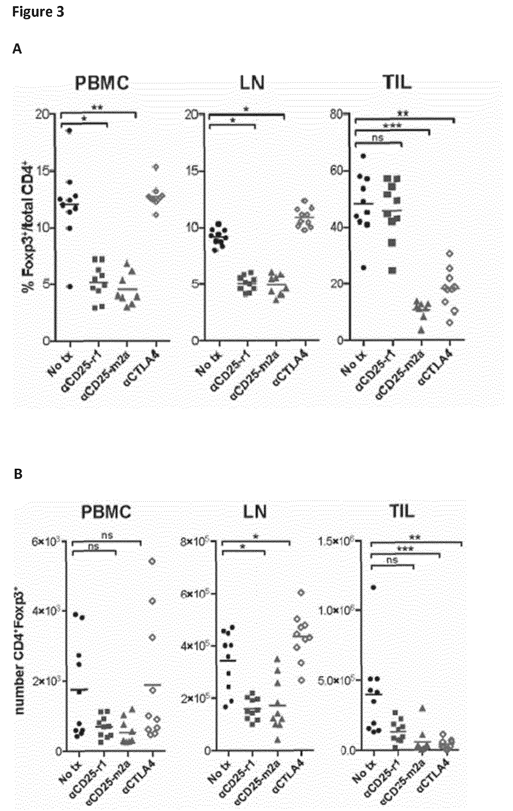

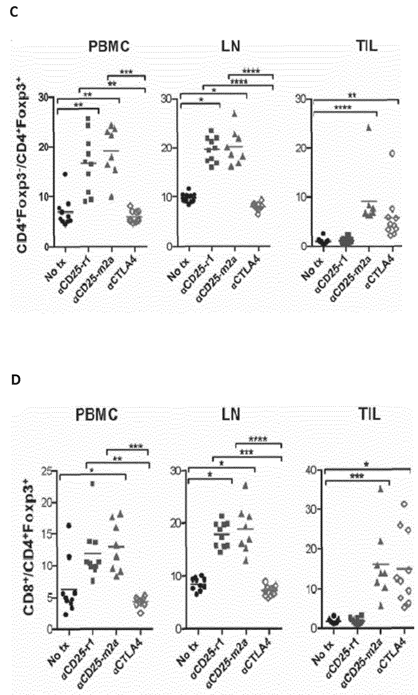

**Figure 3** - shows the anti-CD25 (αCD25)-mediated effects on T cells sub-populations in the MCA205 tumour model of Fig. 2. (A) Percentage of FoxP3-positive cells from total CD4-positive T cells and (B) absolute number of CD4-positive, FoxP3-positive T cells in PBMC (number of cells/mL), LN (total number of cells in three draining lymph nodes) and TIL (number of cells/g of tumour) are shown in parallel to CD4-positive, FoxP3-negative T cells (C) the ratio of effector CD4-positive, FoxP3-positive T cells (Treg cells) and (D) the ratio of effector CD8-positive T cells) / Treg cells, gated on CD4-positive FoxP3-negative and CD8-positive T cells.

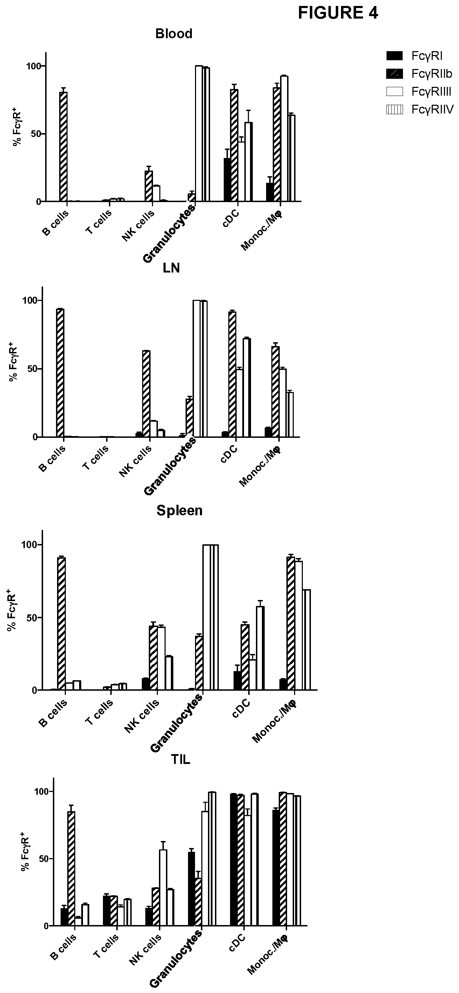

**Figure 4** - shows representative histograms for the expression of FcγRs on B cells (CD19-positive), T cells (CD3-positive, CD5-positive), NK cells (NK1.1-positive), granulocytes (CD11b+ Ly6G+), conventional dendritic cells (cDC; CD11c-high MHCII-positive) and monocyte/macrophages (Mono/Mϕ; CD11b-positive, Ly6G-negative, NK1.1-negative, CD11c-low/negative). as assessed by flow cytometry in untreated MCA205 tumour model (see fig. 2) 10 days after tumour challenge. Error bars represent SEM (n=3); data corresponds to one of three separate experiments across which findings were consistent.

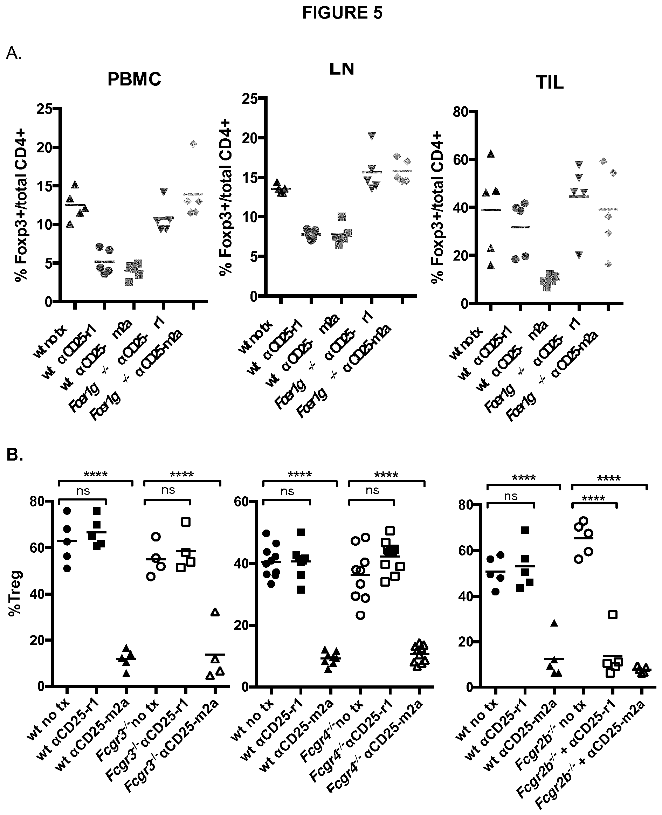

**Figure 5** - shows how Treg depletion depends on expression of activatory Fc-gamma receptors. C57BL/6 wild type mice (wt) and Fcer1g-/- mice were injected subcutaneously with 5 x 10⁵ MCA205 cells on day 0 and then injected with 200 µg of anti-CD25 on days 5 and 7. Tumours, draining lymph nodes and blood were harvested on day 9, processed and stained for flow cytometry analysis. Regulatory T cells were identified by CD4 and FoxP3 expression in PBMC, LN, and TIL. Percentage of Foxp3+ from total CD4+ cells are shown (A). The same approach was applied in wild-type (wt), Fcgr3-/-, Fcgr4-/- or Fcgr2b-/-, demonstrating the inhibition of αCD25-r1-mediated Treg depletion in tumors by FcγRIIb. The plots show quantification of the percentage of Treg (CD4+ Foxp3+) from total CD4+ T cells in TIL only (B).

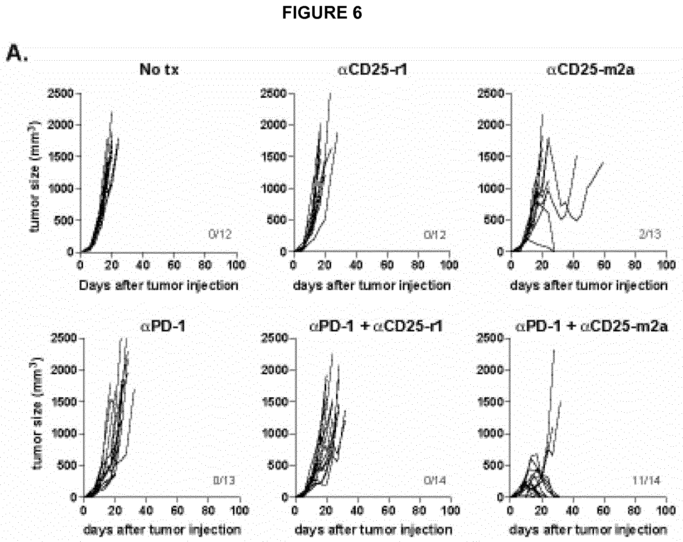

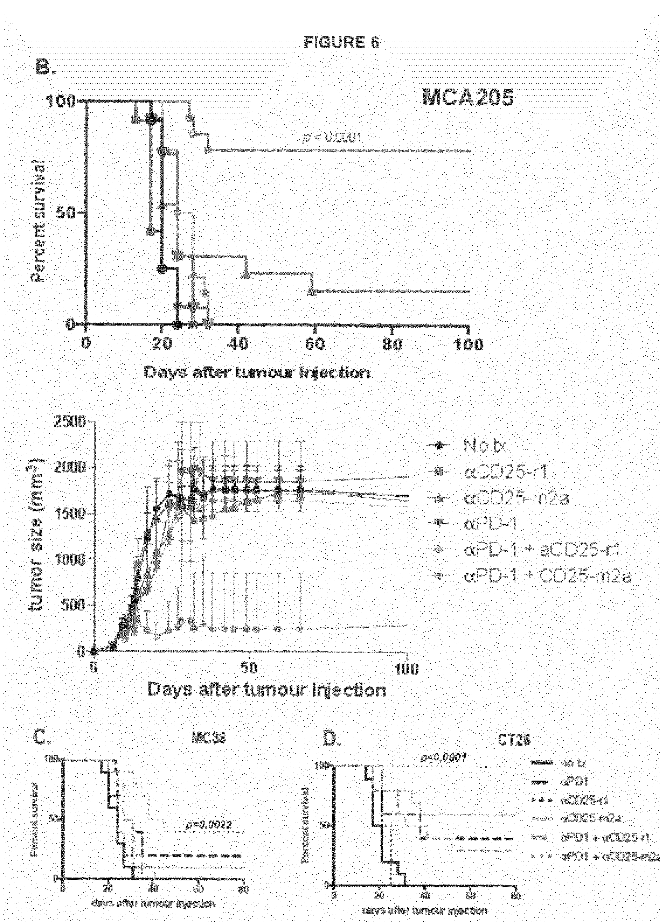

**Figure 6** - shows the synergistic effect of anti-CD25-m2a and anti-PD-1 combination results in eradication of established tumours. Growth curves of individual mice (A) and mean of MCA205 tumour volume for each treatment group over time (B) are shown. The number of tumor-free survivors after 100 days or the statistical significance is indicated in each graph. Error bars represent SE of the mean. Kaplan-Meier survival curves with cumulative data of two separate experiments are also shown. Survival curves of mice injected with MC38 (C) or CT26 (D) tumour cells and treated as described in the MCA205 model (n=10 per condition) are also shown. Mice were injected subcutaneously with 5 x 10⁵ MCA205, MC38, or CT26 cells and then treated with the indicated anti-CD25 (200 µg i.p.) on day 5, followed (or not) by the administration of anti-PD-1 (αPD-1, anti-PD1, clone RMP1-14; 100 µg i.p.) on days 6, 9 and 12. Tumour size was measured twice a week and mice were euthanized when any orthogonal diameter reached 150 mm.

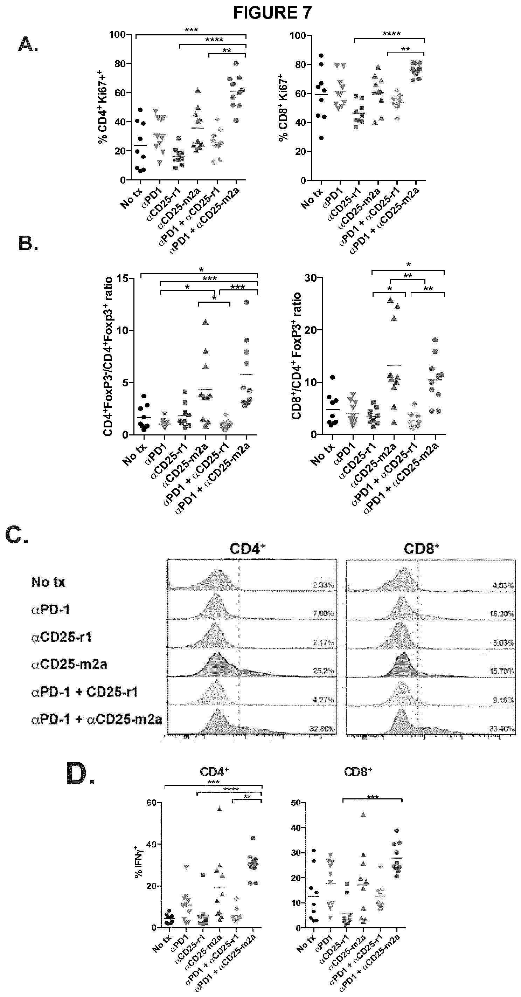

**Figure 7** - shows functional analyses of MCA205 tumour model that was established as described in Fig. 6, using immune cells that were harvested on day 14. Proportion (%) of Ki67+ cells in tumour-infiltrating CD4+Foxp3- and in CD8+ T cells in MCA205 tumours. (A) and the CD4-positive, FoxP3-negative Teff/Treg ratio and CD8-positive/Treg ratio (B) in the tumour is shown for each treatment group. The intracellular staining of tumour-infiltrating lymphocytes for IFNg expression following ex vivo re-stimulation with PMA and ionomycin (C) and frequency of interferon gamma (IFNγ)-producing effector T cells (D) is also shown for the same treatment groups in CD4-positive and CD8-positive cells. Histograms in (B) correspond to a representative mouse per treatment group. Representative plots from two separate experiments (n=10) and statistical significance are provided in (A), (B), and (D).

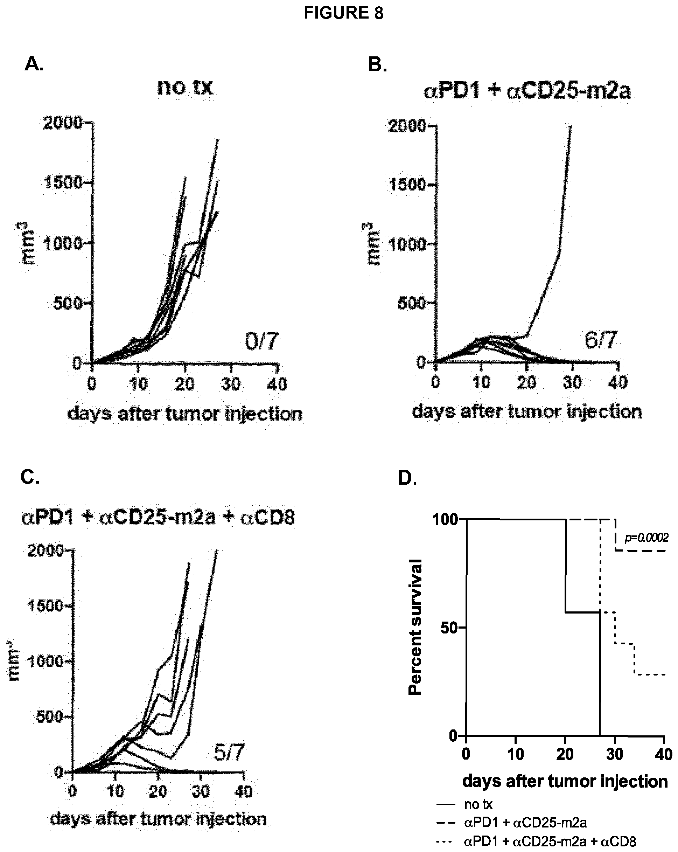

**Figure 8** - shows that tumour elimination by anti-CD25-m2a/anti-PD1 is CD8+ T cell dependent. MCA205 tumour growth curves of individual mice not treated (no tx, A), treated with a combination of anti-CD25-m2a with anti-PD-1 (αPD-1 + αCD25-m2a; B), or the same combination further including anti-CD8 (αPD-1 + αCD25-m2a + αCD8; C). The number of survivors after 40 days for each treatment group (n=7) is indicated in each graph. The corresponding Kaplan-Meier survival curves were also generated (D). Mice were injected s.c. with 5 x 10⁵ MCA205 cells and treated with 200 µg of anti-CD25-m2a (αCD25-m2a, clone PC61, mouse IgG2a isotype) on day 5 followed by 100 µg of anti-PD-1 (αPD-1, clone RMP1-14) i.p. on days 6, 9 and 12. In the indicated group of mice, CD8-positive cells were depleted by injecting 200 µg of anti-CD8 (αCD8, clone 2.43) i.p. on days 4, 9, 12 and 17. Tumour sized was measured twice a week and mice were euthanized when any orthogonal diameter reached 150 mm.

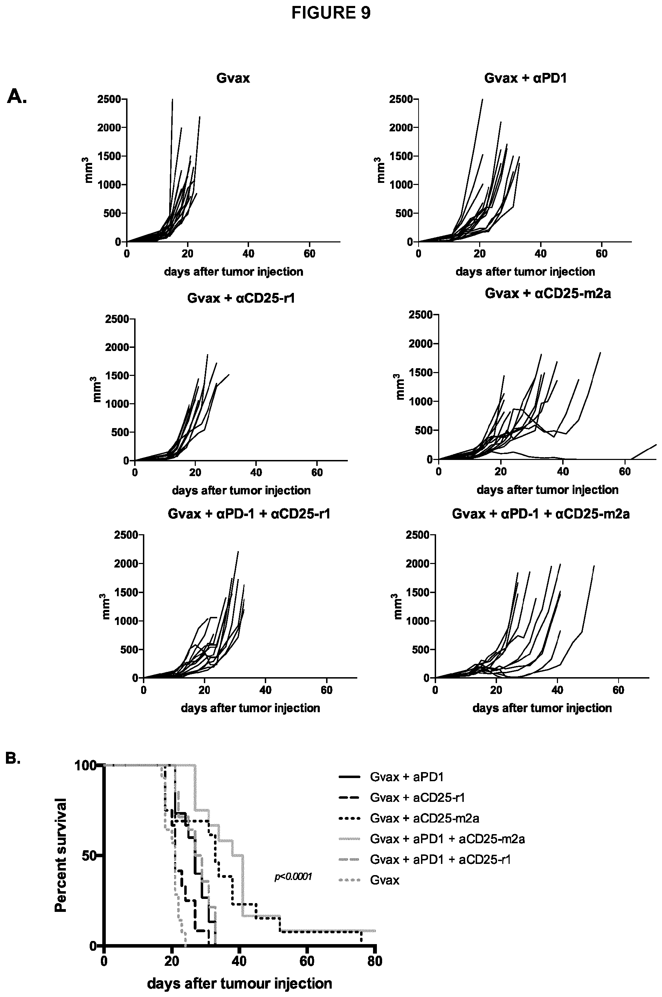

**Figure 9** - shows that anti-CD25-m2a/anti-PD-1 therapy induces at least partial tumour control against B16 melanoma tumours. B16 tumour growth curves of individual mice treated with Gvax alone or in combination with the indicated antibodies, as defined in the figure 6 (A). The corresponding Kaplan-Meier survival curves were also generated (B). Mice were injected with 5 x 10⁴ B16 melanoma cells intra-dermally (i.d.) and then treated with 200 µg of anti-CD25 (αCD25-r1, clone PC61 rat IgG1 isotype or αCD25-m2a, clone PC61 mouse IgG2a isotype) on day 5 followed by 200 µg of anti-PD-1 (αPD-1, clone RMP1-14) i.p. and 1 x 10⁶ irradiated (150 Gy) B16-Gvax i.d. on days 6, 9 and 12. Tumour growth was followed up and the mice euthanized when any orthogonal diameter reached 150 mm or on day 80 of the study, whichever came first. The median survival in days for the different groups (n, number of mice) was: 21d for Gvax only (n=14), 27d for Gvax + αPD-1 (n =15), 21d for Gvax + αCD25-r1 (n=7), 33d for Gvax + αCD25-m2a (n=8), 29d for Gvax + αPD-1 + αCD25-r1 (n =13), and 39d for for Gvax + αPD-1 + αCD25-m2a (n =12).

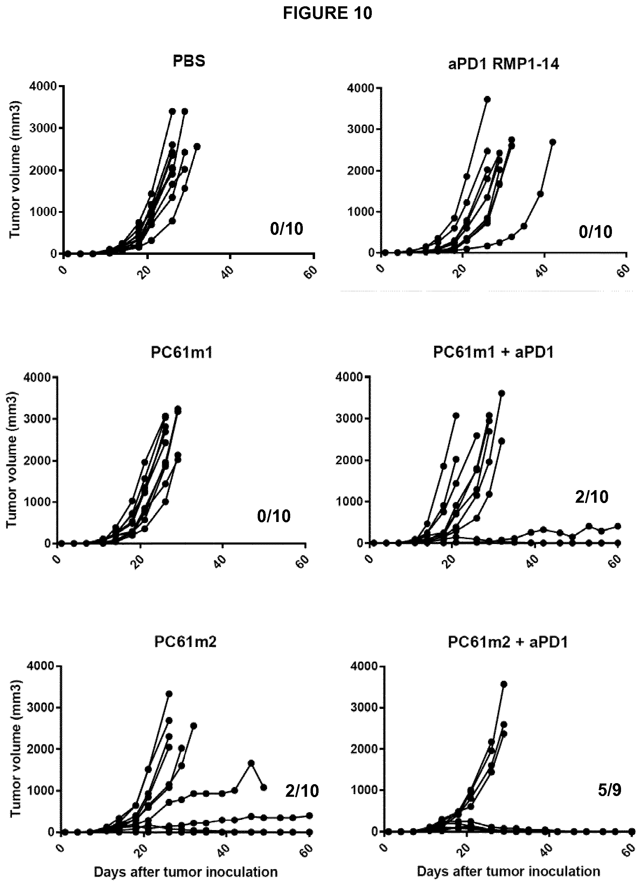

**Figure 10** - shows CT26 tumour growth curves of individual mice not treated (PBS, vehicle only), treated with anti-mouse CD25 having either IgG1 (PC61m1; mouse IgG1 isotype, thus with low FcReceptor-mediated killing activity, low ADCC and CDC activity) or IgG2a (PC61m2; mouse IgG2a, thus with high Fc Receptor mediated activity, high ADCC, and CDC activity), and further combined or not with anti-mouse PD1 (αPD1 RMP1-14). CT26 cells used for implantation were harvested during log phase growth and re-suspended in cold PBS. On Day 1 of the study, each mouse was injected subcutaneously in the right flank with 3 x 10⁵ cells in 0.1 mL cell suspension. The anti-mouse CD25 was injected i.p. (10mg/kg) at Day 6 (when palpable tumours were detected). The anti-mouse PD1 was injected i.p. (100µg/injection) at Day 7, Day 10, Day 14, and Day 17. Tumours were calipered in two dimensions twice weekly to monitor growth. Tumour size, in mm³, was calculated as follows: Tumour Volume = (w2 x I)/2 where w = width and l = length, in mm, of the tumour. The study endpoint was a tumour volume of 2000 mm³ or 60 days, whichever came first.

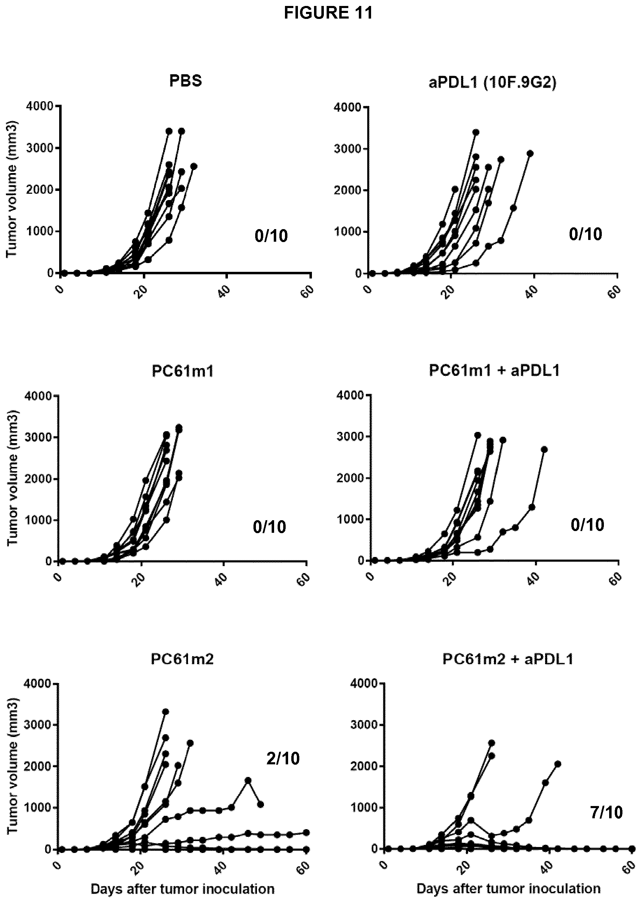

**Figure 11** - shows CT26 tumour growth curves of individual mice not treated (PBS, vehicle only), treated with anti-mouse CD25 having either IgG1 or IgG2a (PC61m1, and PC61m2 with, respectively), and further combined or not with anti-mouse PD-L1 clone 10F.9G2 (aPDL1 10F.9G2). Model, regimen, and analysis was performed as for the αPD1-based combination experiment of Fig. 10.

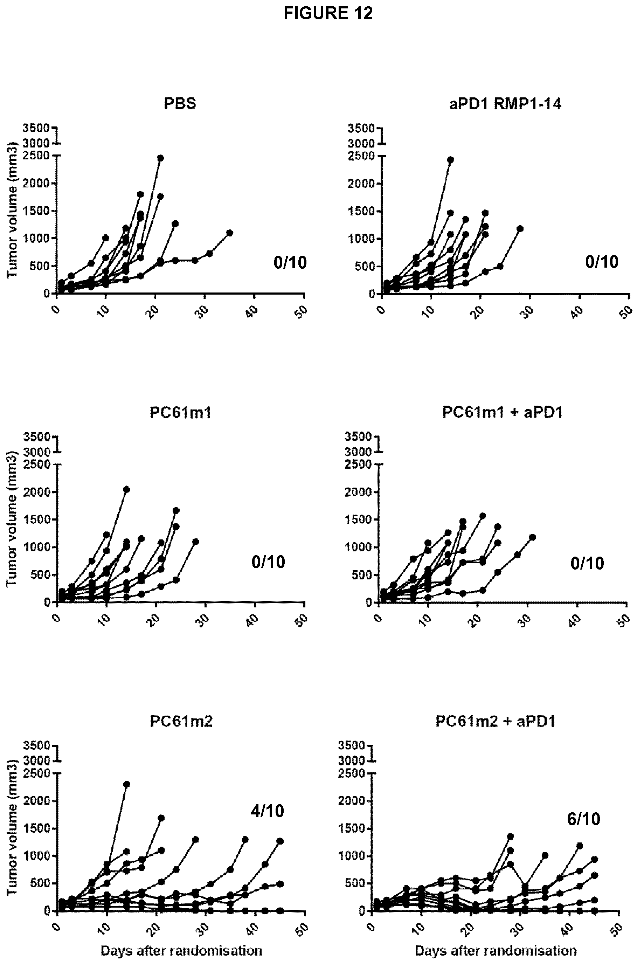

**Figure 12** - shows MC38 tumour growth curves of individual mice not treated (PBS, vehicle only), treated with anti-mouse CD25 having either IgG1 or IgG2a (PC61m1, and PC61m2, respectively), and further combined or not with anti-mouse PD1 clone RMP1-14 (aPD1 RMP1-14), as described for CT26 tumour model in Fig. 10. The MC38 colon carcinoma cells used for implantation were harvested during log phase growth and re-suspended in cold PBS. Each mouse was injected subcutaneously in the right flank with 5 x 10⁵ tumour cells in a 0.1 mL cell suspension. Tumours were monitored as their volumes approached the target range of 100 to 150 mm³. Twenty-two days after tumour implantation, on Day 1 of the study, animals with individual tumour volumes ranging from 63 - 196 mm³ were sorted into nine groups (n = 10) with group mean tumour volumes ranging from 104 - 108 mm³. Treatments began on D1 in mice bearing established MC38 tumours. The effects of each treatment were compared to a vehicle-treated control group that received PBS intraperitoneally (i.p.) on Day 1, Day 2, Day 5, Day 9, and Day 12. Anti-PD1 was administered i.p. at 100 µg/animal, twice weekly for two weeks, beginning on Day 2. PC61-m1 and PC61-m2a were administered i.p. once on Day 1 at 200 µg/animal. Tumour measurements were taken twice weekly until Day 45 with individual animals exiting the study upon reaching the tumour volume endpoint of 1000 mm³.

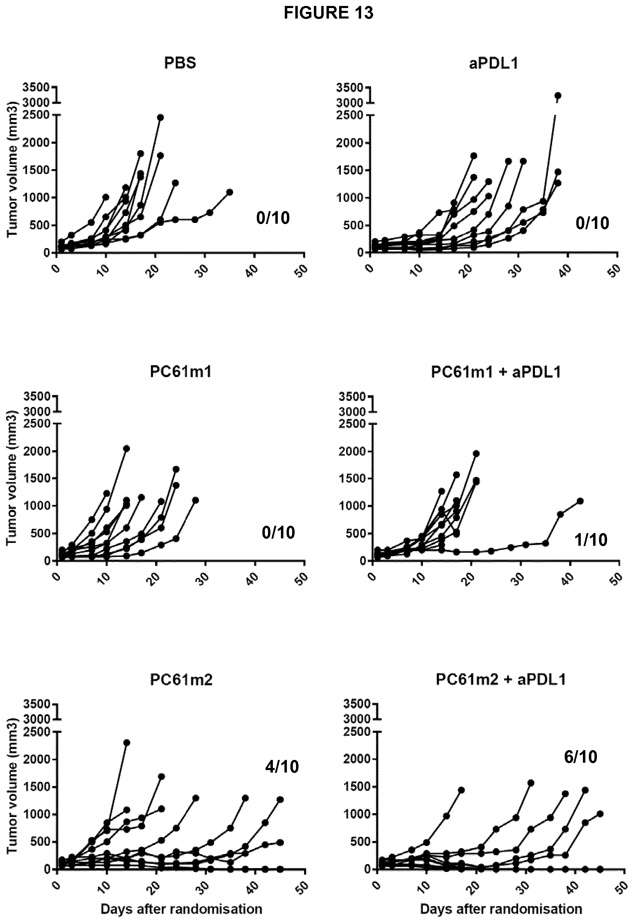

**Figure 13** -shows MC38 tumour growth curves of individual mice not treated (PBS, vehicle only), treated with anti-mouse CD25 having either IgG1 or IgG2a (PC61m1, and PC61m2 with, respectively), and further combined or not with anti-mouse PD-L1 clone 10F.9G2 (aPDL1 10F.9G2). Model, regimen, and analysis was performed as for the αPD1-based combination experiment of Fig. 12.

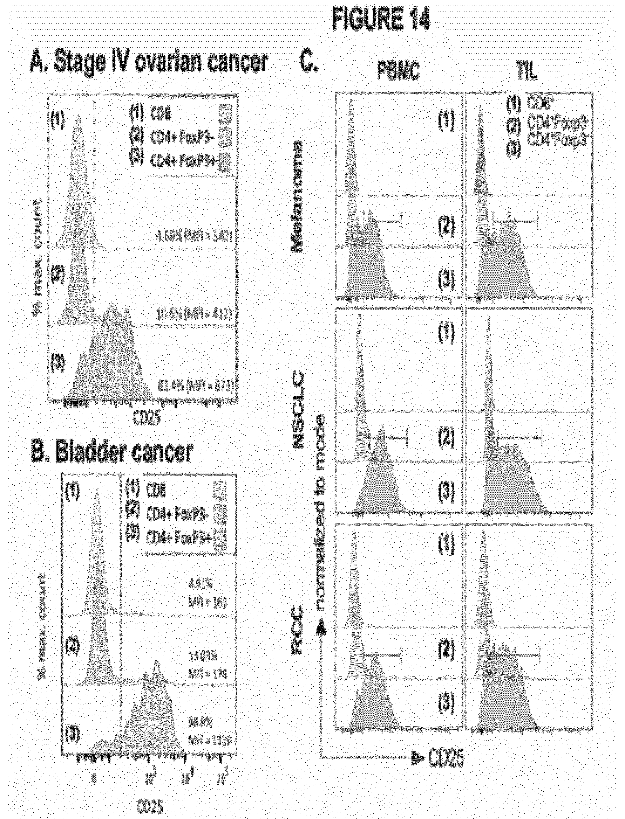

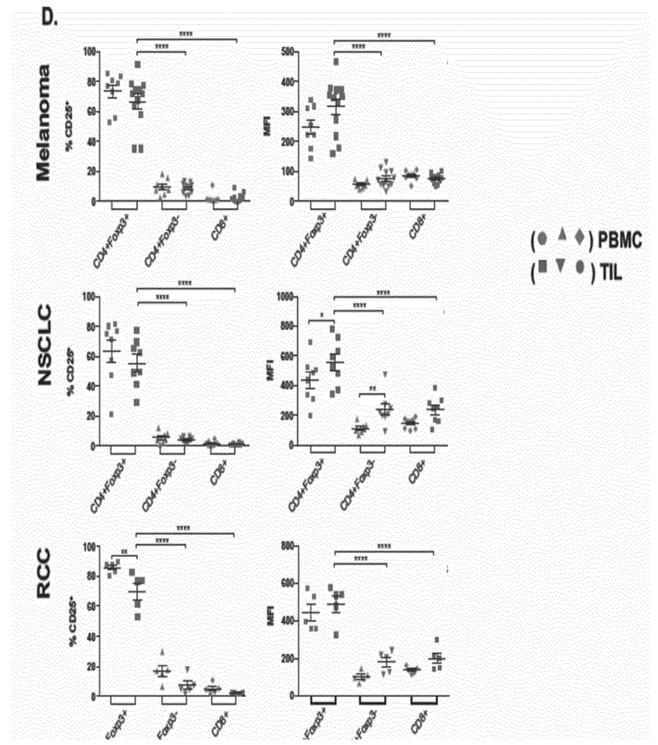

**Figure 14** - shows CD25 expression in peripheral of tumour-localized immune cells in samples from distinct types of human cancers. Representative histograms demonstrate CD25 expression in TIL subsets from a stage IV human ovarian carcinoma (peritoneal metastasis; A) and in a human bladder cancer (B). Representative histograms were also obtained for individual CD8-positive, CD4-positive, FoxP3-negative and CD4-positive, FoxP3-positive T cell subsets within PBMC and TIL that are isolated from other types of cancer (C). Quantification of CD25 expression as percentage (%) and mean fluorescence intensity (MFI) on individual T cell subsets within each studied patient cohort for melanoma (upper panel), NSCLC (middle panel) and RCC (lower panel) is also shown (D).

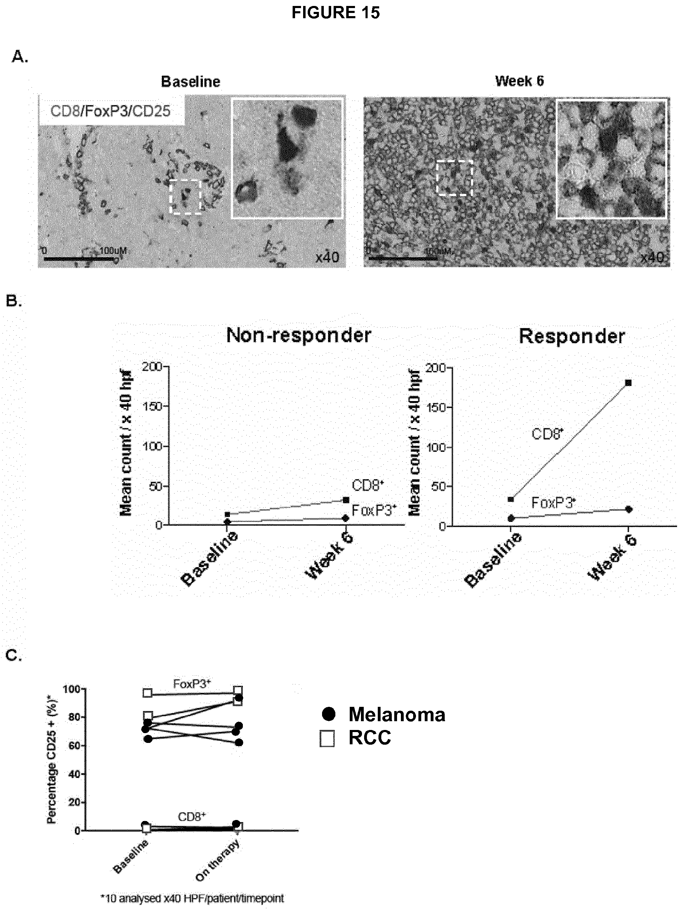

**Figure 15** - shows data on CD25 expression in patients treated with anti-PD-1 Multiplex immunohistochemical (IHC) analysis of a subcutaneous melanoma metastasis prior to anti-PD-1 therapy ('Baseline') and following two infusions ('Week 6') is shown in parallel to the quantification of CD8 and FoxP3 IHC staining at baseline and week 6 in two patients, one responding and one non-responding to therapy at week 6 (B; mean count per x 40 high power field is displayed). Percentage (%) of CD8-positive, CD25-positive, and FoxP3-positive, CD25-positive, double-stained cells at baseline and on therapy (at week 6) is shown for Melanoma and RCC patients treated with anti-PD1 (C).

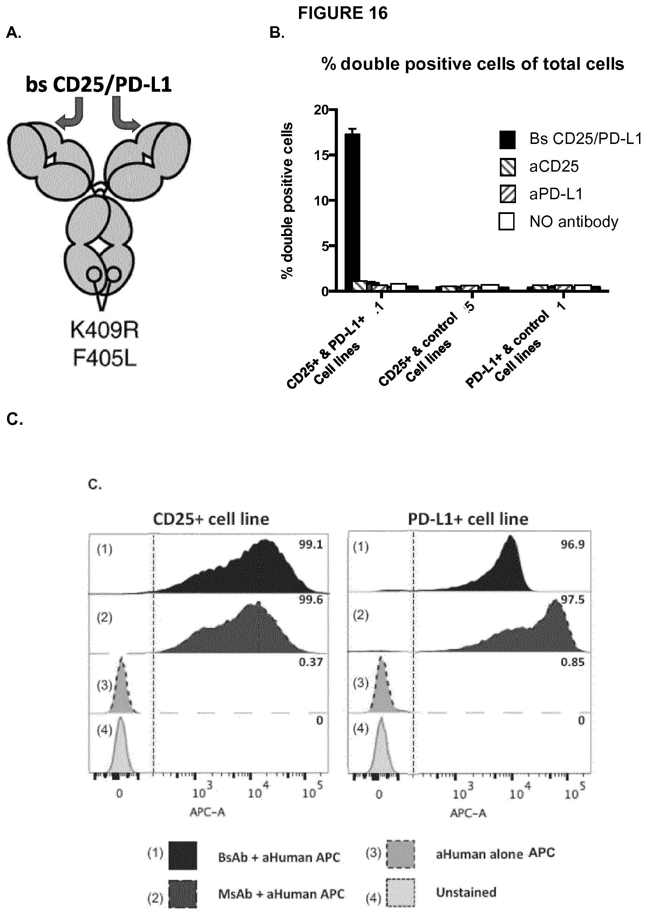

**Figure 16** - shows structure and binding activity of bispecfiic anti-lgG1-, anti-PD-L1-based Duobody (bs CD25/PD-L1) that have been generated by using the antigen binding region of anti-mouse CD25 (PC61) and anti-mouse/human PD-L1 (clone S70), both having a human IgG1 isotype and mutated in a specific amino acid (K409R for PC61-IgG1 and F405L for S70-IgG1; A). The specificity of bs CD25/PD-L1 for CD25 have been tested using a cell line (SUP-T1 cells, human T lymphoblasts; SUP-T1 [VB] ATCC^{®} CRL-1942^{™}) that has been transfected with a vector expressing either mouse CD25 (CD25+ cell line) or mouse PD-L1 (PD-L1+ cell line). The original cell line and the other two resulting cell lines have been used to compare the binding ability of bs CD25/PD-L1 to binding of the related monospecific antibody (aCD25, clone PC61; aPD-L1, clone S70). The CD25+ cell line and PD-L1+ cell lines are mixed at 1:1 ratio with each other (or each separately with untransfected, control cells), then incubated with bs CD25/PD-L1, aCD25, aPD-L1, or without any antibody (NO antibody) for 30 minutes. After the incubation, the three groups of cell samples are analysed in flow cytometry to calculate the percentage of double positive cells in the different cell samples (B). The specificity of bs CD25/PD-L1 (BsAb) has been confirmed using the CD25+ cell line and PD-L1+ cell lines separately. Each cell line have been labelled with either the bs CD25/PDL1, or the respective monospecific Ab (MsAb, anti-mouse CD25 IgG1 for CD25+ cells and anti-mouse PD-L1 for PD-L1+ cells) as primary antibody, or with buffer only. Cells were then incubated with aHuman AF647 (aHuman) as secondary antibody in FACS buffer for 30 mins as well as fixable viability dye. Cells incubated with the secondary antibody only (aHuman AF647) or cells incubated with neither primary nor secondary antibody (unstained) are used as negative controls. Cells are then analysed by flow cytometry to calculate the percentage of positive cells obtained with BsAb compared to MsAb (indicated in the right of each panel; C).

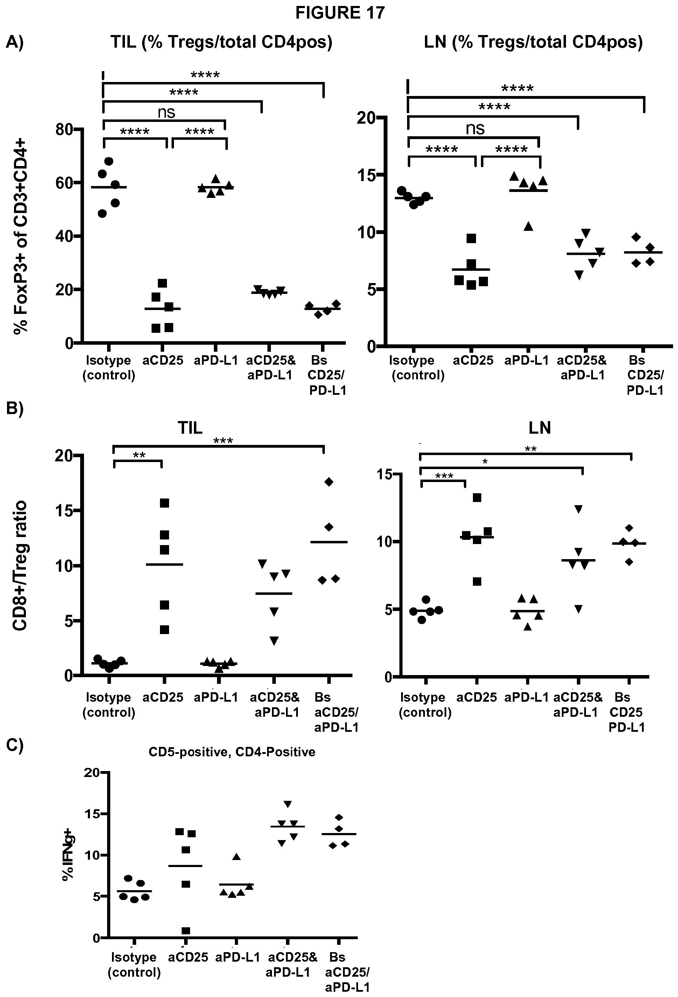

**Figure 17** - shows the impact of bispecific IgG1-based Duobody (Bs CD25 PD-L1), the anti-mouse CD25 (aCD25) IgG1 and the anti-mouse PD-L1 (aPD-L1) IgG1 monospecific antibodies, separately or mixed together (aCD25&aPD-L1), or isotype IgG1 control, as described in Fig. 16, on effector and regulatory T cells in LN and tumour in the MCA205 tumour mouse model (established as described in Fig.3; with four or five mice for each group). The samples were used for isolating Tumor Infiltrating Lymphocytes (TIL) or in lymph node cells (LN) were isolated and analysed for the presence of effector and regulatory T cells th in each treatment group on the basis of FoxP3, CD3, and CD4 positivity (A) or of CD8 positivity / Treg (FoxP3-positive, CD4-positive) ratio (B). The in vivo effect of each treatment on the ability of tumor-infiltrating CD4-positive T cells to respond to stimulation was also evaluated. TIL were re-stimulated in vitro using PMA and ionomycin, in the presence of a Golgi plug protein inhibitor and then stained extracellularly for CD5 and CD4 and intracellularly after fixation for Interferon-y (IFNg). The percentage of CD5-positive and CD4-positive T cells also positive for IFNg was analysed by flow cytometry (C).

### EXAMPLES

### MATERIALS & METHODS

### Mice

C57BL/6 and BALB/c mice were obtained from Charles River Laboratories. *Fcer1g^{-l-}* and *Fcgr3*^{*-*/*-*} mice were kindly provided by S. Beers (University of Southampton, UK). *Fcgr4*^{*-*/*-*} and *Fcgr2b*^{*-*/*-*} mice were a kind gift from J.V. Ravetch (The Rockefeller University, New York, USA). All animal studies were performed under University College of London and UK Home Office ethical approval and regulations.

### Cell lines and tissue culture

MC38, B16, CT26, and MCA205 tumour cells (3-methylcholanthrene-induced weakly immunogenic fibrosarcoma cells; from G. Kroemer, Gustave Roussy Cancer Institute) and 293T cells used for retrovirus production were cultured in Dulbecco's modified Eagle medium (DMEM, Sigma) supplemented with 10% fetal calf serum (FCS, Sigma), 100 U/mL penicillin, 100 µg/mL streptomycin and 2 mM L-glutamine (all from Gibco). K562 cells used for antibody production were cultured in phenol red-free Iscove modified Dulbecco medium (IMDM) supplemented with 10% IgG-depleted FCS (Life Technologies). B16 (mouse skin melanoma cells) and CT26 (N-nitroso-N-methylurethane-induced, undifferentiated colon carcinoma cell line) cells and related culture conditions are available through ATCC.

### Antibody production

The sequence of the variable regions of the heavy and light chains of anti-CD25 were resolved from the PC-61.5.3 hybridoma by rapid amplification of cDNA ends (RACE) and then cloned into the constant regions of murine IgG2a and κ chains sourced from the pFUSEss-CHIg-mG2A and pFUSE2ss-CLIg-mk plasmids (Invivogen). Each antibody chain was then sub-cloned into a murine leukemia virus (MLV)-derived retroviral vector. For preliminary experiments, antibody was produced using K562 cells transduced with vectors encoding both the heavy and the light chains.

The re-cloned, anti-CD25 heavy variable DNA sequence from PC-61.5.3 antibody encodes for the following protein sequence:

METDTLLLWVLLLWVPGSTGEVQLQQSGAELVRPGTSVKLSCKVSGDTITAYYIHFV
KQRPGQGLEWIGRIDPEDDSTEYAEKFKNKATITANTSSNTAHLKYSRLTSEDTATY
FCTTDNMGATEFVYWGQGTLVTVSS

The re-cloned, anti-CD25 light variable DNA sequence from PC-61.5.3 antibody encodes for the following protein sequence:

METDTLLLWVLLLWVPGSTGQVVLTQPKSVSASLESTVKLSCKLNSGNIGSYYMHW
YQQREGRSPTNLIYRDDKRPDGAPDRFSGSIDISSNSAFLTINNVQTEDEAMYFCHS
YDGRMYIFGGGTKLTVL

The antibody was purified from supernatants using a protein G HiTrap MabSelect column (GE Healthcare), dialyzed in phosphate-buffered saline (PBS), concentrated and filter-sterilized. For further experiments, antibody production was outsourced to Evitria AG. Commercial anti-CD25 clone PC-61 was purchased from BioXcell.

The published anti-PDL1 (MPDL3280A/RG7446 ) variable heavy and light DNA sequences have been recloned and expressed as recombinant antibodies.

### In vivo tumour experiments

Cultured tumour cells were trypsinized, washed and resuspended in PBS and injected subcutaneously (s.c.) in the flank (5 x 10⁵ cells for MCA205 and MC38 models in C57BL/6 mice; 2.5 x 10⁵ cells for B16 model in C57BL/6 mice, 5 x 10⁵ cells for CT26 models in BALB/c mice) cells). Antibodies were injected intraperitoneally (i.p.) at the time points described in the figure legends. For functional experiments, 10 days later the tumors, draining lymph nodes, and tissues were harvested and processed for analysis by flow cytometry as described in Simpson et al. (2013) J Exp Med 210, 1695-710. For therapeutic experiments, tumours were measured twice weekly and volumes calculated as the product of three orthogonal diameters. Mice were humanely euthanized when any diameter reached 150 mm. Tumor-bearing mice were treated with 200 µg of anti-CD25-r1 (αCD25-r1), anti-CD25-m2a (αCD25-m2a) or anti-CTLA-4 (αCTLA-4) on days 5 and 7 and 100 µg of anti-PD-1 on days 6, 9 and 12. For the therapeutics experiments, the mice were only treated on day 5, for the phenotyping and depletion on day 5 and 7. Tumour size was measured twice a week and mice were euthanized when any tumour dimension reached 150 mm. Peripheral blood mononuclear cells (PBMC), lymph nodes (LN) and tumors (TIL) were harvested on day 9, processed and stained for flow cytometry analysis.

### Flow cytometry

Acquisition was performed with a BD LSR II Fortessa (BD Biosciences). The following directly conjugated antibodies were used: anti-CD25 (7D4)-FITC, CD4 (RM4-5)-v500 (BD Biosciences); anti-IFNγ (XMG1.2)-AlexaFluor488, anti-PD-1 (J43)-PerCP-Cy5.5, anti-Foxp3 (FJK-16s)-PE, anti-CD3 (145-2C11)-PE-Cy7, anti-Ki67 (SolA15)-eFluor450, anti-CD5 (53-7.3)-eFluor450, fixable viability dye-eFluor780 (eBioscience); anti-CD8 (53-6.7)-BrilliantViolet650 (BioLegend); and anti-granzyme B (GB11)-APC (Invitrogen). The following antibodies were used to stain human cells: anti-CD25 (BC96)-BrilliantViolet650 (Biolegend), anti-CD4 (OKT4)-AlexaFluor700 (eBioscience), anti-CD8 (SK1)-V500, anti-Ki67 (B56)-FITC (BD Biosciences); anti-FoxP3 (PCH101)-PerCP-Cy5.5 (eBioscience); anti-CD3 (OKT3)-BrilliantViolet785 (Biolegend). Intranuclear staining of Foxp3 was done using the Foxp3 Transcription Factor Staining Buffer Set (eBioscience). For intracellular staining of cytokines, cells were re-stimulated with phorbol 12-myristate 13-acetate (PMA, 20 ng/mL) and ionomycin (500 ng/mL) (Sigma Aldrich) for 4 hours at 37C in the presence of GolgiPlug (BD Biosciences) and then stained using Cytofix/Cytoperm buffer set (BD Biosciences). For quantification of absolute number of cells, a defined number of fluorescent beads (Cell Sorting Set-up Beads for UV Lasers, ThermoFisher) was added to each sample before acquisition and used as counting reference.

### Human tissues

Peripheral blood (PBMCs) and tumor-infiltrating lymphocytes (TIL) were studied in three separate cohorts of patients with advanced melanoma (n=10, 12 lesions), early-stage non-small cell lung cancer (NSCLC) (n=8) and renal cell carcinoma (RCC) (n=5). Presented human data derives from three separate, ethically approved, translational studies (melanoma REC no. 11/LO/0003, NSCLC - REC no.13/LO/1546, RCC-REC no. 11/LO/1996). Written, informed consent was obtained in all cases.

### Isolation of tumour-infiltrating lymphocytes (TILs)

Tumours were taken directly from the operating theatre to the department of pathology, where tumour representative areas were isolated. Samples were subsequently minced under sterile conditions followed by enzymatic digestion (RPMI-1640 (Sigma) with Liberase TL research grade (Roche) and DNAse I (Roche)) at 37°C for 30 minutes before mechanical dissociation using gentleMACS (Miltenyi Biotech). Resulting single cell suspensions were filtered and enriched for leukocytes by passage through a Ficoll-paque (GE Healthcare) gradient. Live cells were counted and frozen in human AB serum (Sigma) with 10% dimethyl sulfoxide at -80°C before transfer to liquid nitrogen.

### Phenotypic analysis of TILs and PBMCs by multi-parametric flow cytometry

Tumour samples and PBMCs were thawed, washed in complete RPMI, re-suspended in FACS buffer (500mL PBS, 2% FCS, 2nM EDTA) and placed in round-bottomed 96 well plates. A mastermix of surface antibodies was prepared at the manufacturer's recommended dilution: CD8-V500, SK1 clone (BD Biosciences), PD-1-BV605, EH12.2H7 clone (Biolegend), CD3-BV785,. A fixable viability dye (eFlour780, eBioscience) was also included the surface mastermix. Following permeablisation for 20 minutes with use of an intracellular fixation and permeabilization buffer set (eBioscience), an intracellular staining panel was applied consisting of the following antibodies used at the manufacturers recommended dilution: granzyme B-V450, GB11 clone (BD Biosciences), FoxP3-PerCP-Cy5.5, PCH101 clone (eBioscience), Ki67-FITC, clone B56 (BD Biosciences) and CTLA-4 - APC, L3D10 clone (Biolegend).

### Multiplex immunohistochemistry

Tumour samples were fixed in buffered formalin and embedded in paraffin. 2-5µm tissue sections were cut and stained with the following antibodies for immunohistochemistry: anti-CD8 (SP239), anti-CD4 (SP35) (Spring Biosciences Inc.), anti-FoxP3 (236A/E7) (a gift from Dr. G. Roncador CNIO, Madrid, Spain) and anti-CD25 (4C9) (Leica Biosystems). For multiple staining, paraffin-embeded tissue sections were incubated with the primary antibodies for 30 min after antigen retrieval by using cell conditioning 1 reagent (Ventana Medical Systems, Inc.) and hydrogen peroxide for inactivation of endogenous peroxidase. Detection was performed using a peroxidase-based detection reagent (OptiView DAB IHC Detection Kit Ventana Medical Systems, Inc.) and an alkaline phosphatase detection reagent (UltraView Universal Alkaline Phosphatase Red Detection Kit, Ventana Medical Systems, Inc.). A further cycle of immuno-alkaline phosphatase was performed by using an alternative substrate (Fast Blue if Fast Red had been used previously, or vice versa). Immunohistochemistry and protein reactivity patterns were assessed. Scoring of multiple immuno-staining was also performed. Approval for this study was obtained from the National Research Ethics Service, Research Ethics Committee 4 (REC Reference number 09/H0715/64).

### Construction and validation of Anti-CD25- and anti-PD-L1-based bispecific Duobody

The Bs CD25 PD-L1 Duobody has been generated and produced in accordance to technology described in the literature starting from two parental IgG1s containing single matching point mutations in the CH3 domain that allow Fab exchange (Labrijn AF et al., Nat Protoc. 2014, 9:2450-63). Briefly, each of the anti-mouse CD25 (PC61; mouse IgG1 isotype, as described above) and the anti-mouse/human PD-L1 (clone S70, also known as Atezolizumab, MPDL3280A, RG7446, or clone YW243.55.S70; see WO2010077634 and Herbst R et al., 2014, Nature 515:563-7) is cloned in mammalian expression vectors (504865 | UCOE^{®} Expression Vector - Mouse 3.2 kb Puro Set - Novagen) with K409R mutation (for PC61-IgG1) and F405L mutation (for S70-IgG1) in CH3 domain, while light chains are maintained identical, and produced as separate recombinant proteins in mammalian cells. These parental IgG1s are mixed in vitro in equimolar amounts, under permissive redox conditions (e.g. 75mM 2-MEA; 5h incubation) in order to enable recombination of half-molecules. Following the removal of the reductant to allow reoxidation of interchain disulfide bonds, resulting heteromeric proteins are analysed for exchange efficiency using SDS-PAGE chromatography-based or mass spectrometry-based methods. In the case of Bs CD25 PD-L1, the mass spectrometry has confirmed that the molecular weight of the heterodimeric proteins was 151 Kd, corresponding to the addition of the molecular weight of Clone S70 single Heavy Chain and Light Chain (74 Kd) and PD61-IgG1 single Heavy Chain and Light Chain (77 Kd) and showing that half of each parental IgG1 have been combined in a single molecule.

The specificity of Bs CD25 PD-L1 has been further confirmed by flow cytometry as described in Example 5, using the parental antibodies as control, and IgG1-recognizing detections antibodies (aHuman, Alexa Fluor^{®} 647, AffiniPure Goat Anti-Human IgG, Fcγ Fragment Specific; Jackson Labs 109-605-098), that are used according to literature and manufacturer's instructions diluted in FACS buffer (PBS + 2% FCS + 2mM EDTA). Additional flow cytometry and cell biology materials are fixable viability dye eFluor780 (Ebioscience 65086514), PMA (50ng/ml; Santa Cruz Biotechnology, sc-3576) and ionomycin (400ng/ml; Sigma I0634), and Golgi plug protein inhibitor (BD Bioscience, 512301KZ).

The validation of Bs CD25 PD-L1 in MCA205 models has been performed by using the same approach shown in previous Examples, with isotype control, monospecific antibodies (100 µg each) or bispecific Duobody (200 µg each) administered at day 7 after MCA205 injection and mouse tissue obtained and prepared at day 10.

### EXAMPLE 1 - High expression of CD25 in Treg makes it a suitable target for their depletion

The interleukin-2 high affinity receptor alpha (IL2Rα), CD25, has historically been used as a bona fide surface marker of Treg and therefore a target for antibody-mediated Treg depletion. Because there has been controversy as to whether anti-CD25 (aCD25) can also result in elimination of activated effector T cells, the expression of CD25 was analysed in lymphocyte subpopulations in tumours and peripheral lymphoid organs.

Mice were injected subcutaneously (s.c.) in the flank with MCA205 (5 x 10⁵ cells, C57BL/6 mice), B16 (2.5 x 10⁵ cells, C7BL/6 mice) or CT26 (5 x 10⁵ cells, BALB/c mice) cells and 10 days later the tumours (TIL) and draining lymph nodes were harvested and processed for analysis by flow cytometry.

We sought to evaluate the relative expression of CD25 by individual T lymphocyte subpopulations within tumours, draining lymph nodes and the blood of tumour-bearing mice 10 days after tumour challenge. The results are shown in Figure 1. Across different models of transplantable tumour cell lines (including MCA205 sarcoma, MC38 colon adenocarcinoma, B16 melanoma and CT26 colorectal carcinoma), CD25 expression was consistently high in CD4-positive, Foxp3-positive T cells (Treg) and minimal in CD4+Foxp3- and CD8+ T cells (Fig. 1 (A)), as has been previously described (Sakaguchi et al. 1995. J Immunol; Shimizu et al. 1999. J Immunol) . Because of its immunogenicity and higher T cell infiltration, the effects on Treg depletion in the MCA205 tumour model were studied in more detail (Fig. 1 (B-C)). Contrary to *in vitro* studies, minimal expression of CD25 on the effector compartment (CD4⁺FoxP3⁻ and CD8⁺ T cells) was observed *in vivo.* Although CD25 was slightly upregulated on tumor-infiltrating CD8⁺ and CD4⁺FoxP3⁻ T effector cells (Teff). The percentage of CD25-positive cells (3.08%-8.35% CD8+, 14.11-26.87% CD4-positive, Foxp3-negative cells) and the expression levels on a per cell basis (mean fluorescence intensity (MFI) 166.6 in CD8-positive and 134 in CD4-positive, Foxp3-negative cells) were considerably lower than in Treg (83.66-90.23%, MFI 1051.9; p<0.001). Finally, CD25 was also expressed on the Treg present in draining lymph nodes and blood, although the level of expression based on mean fluorescence intensity (MFI) was higher on tumor-infiltrating Treg. The considerably lower expression of CD25 on Teff cells compared to Treg cells indicate that CD25 is a suitable and attractive target for Treg depletion in the tumour where expression levels on Treg are significantly higher.

### EXAMPLE 2 - Isotype swapping is necessary for the effective and safe intratumoural Treg depletion with anti-CD25

Traditionally, the anti-CD25 antibody (αCD25) clone PC-61 (rat IgG1,κ) (αCD25-r1) has been used for Treg depletion in mouse models, in which it has been repeatedly shown to result in elimination of Treg in peripheral lymphoid organs. To avoid the inter-species differences in FcγR engagement, the constant regions of PC-61 were swapped with the murine IgG2a, κ (αCD25-m2a) - the classical mouse depleting isotype - and the number of Treg both in the periphery and in the tumour were quantified and compared to the effect of anti-CTLA4 (αCTLA4, clone 9H10), which is known to result in depletion of tumour-infiltrating Treg.

Based on previous evidence demonstrating the importance of intra-tumoral Treg depletion in co-defining the activity of immune modulatory antibodies, we sought to compare the effect of αCD25-r1 on the frequency of Teff and Treg in the blood, draining lymph nodes (LN) and tumour-infiltrating lymphocytes (TILs) in the MCA205 mouse model, because of its higher immunogenicity and for evaluating any potential negative impact of anti-CD25 on activated Teff within tumours.

Tumour-bearing mice were injected with 200 µg of anti-CD25-r1 (αCD25-r1), anti-CD25-m2a (αCD25-m2a) or anti-CTLA-4 (αCTLA-4) on days 5 and 7 after s.c. inoculation with 5 x 10⁵ MCA205 cells. Peripheral blood mononuclear cells (PBMC), lymph nodes (LN) and tumours (TIL) were harvested on day 9, processed and stained for flow cytometry analysis. The results are shown in Figures 2 and 3.

*In vivo* administration of αCD25 decreased the number of CD25+ cells in lymph nodes and particularly in blood, independently of the antibody isotype. When quantifying the number of Treg by expression of their signature transcription factor, Foxp3, both isotypes were again equally effective in the periphery but, surprisingly, only the mouse IgG2a isotype resulted in a significant reduction in the frequency and absolute number of tumour-infiltrating Treg to levels comparable to those observed with αCTLA4. Although CD25 expression is upregulated in a small proportion of tumour-infiltrating effector T cells (see Example 1), we observed no significant reduction in the number of CD8+ and CD4+Foxp3- in the periphery or in the tumour. As a consequence, both αCD25 isotypes resulted in an increased Teff/Treg ratio in the periphery. However, only αCD25-m2a increased this ratio in a similar way to anti-CTLA4, which is known to preferentially deplete Treg in the tumour site but not the periphery. This potentially explains the lack of efficacy observed against established tumors in previous studies. Thus, only anti-CD25 (mouse IgG2a) reduces the number of Treg in lymph node and blood and depletes tumour-infiltrating Treg. Importantly, despite a reduction in the number of circulating and LN-resident Treg, no macroscopic, evidence of toxicity was observed in the skin, lungs and liver following multiple doses of αCD25-m2a. This type of anti-CD25 therapy was not associated other major problems due to its toxicity in mice during such experiments, since no statistically relevant differences in the general health status and total body weight, as well in serum levels of lactate dehydrogenase (LDH) and liver enzymes (AST, aspartate aminotransferase; ALT, alanine amino-transferase) were measured among the different treatment groups.

The expression levels of both activatory and inhibitory FcγRs on different leukocyte subpopulations in the blood, spleen, LN and tumor of mice bearing subcutaneous MCA205 tumors was also determined (Fig. 4). FcγRs appeared more expressed on tumor-infiltrating myeloid cells (granulocytic cells, conventional dendritic cells and monocyte/macrophages), relative to all other studied organs. The binding affinity of the two Fc variants of anti-CD25 to FcγRs was also determined by surface plasmon resonance (Table 1).

**Table 1**

| | **rIgG1** | **mIgG2a** |
|---|---|---|
| **FcγRI** | n.b. | 1.1 x 10⁻⁸ |
| **FcγRIIb** | 2.6 x 10⁻⁶ | 4.2 x 10⁻⁶ |
| **FcγRIII** | 2.5 x 10⁻⁶ | 4.5 x 10⁻⁶ |
| **FcγRIV** | n.b. | 2.2 x 10⁻⁷ |

These data demonstrate that mIgG2a isotype binds to all FcγR subtypes with a high activatory to inhibitory ratio (A/I). In contrast, the rIgG1 isotype binds with a similar affinity to a single activatory FcγR, FcγRIII, as well as the inhibitory FcγRIIb, resulting in a low A/I ratio (<1).

The number of tumor-infiltrating Treg in mice lacking expression of different FcγRs was established in different mouse models to distinguish which specific FcγRs were involved in anti-CD25-mediated Treg depletion (Fig. 5). C57BL/6 control mice and Fcer1g-/- mice were injected subcutaneously MCA205 cells and tumours, draining lymph nodes and blood were harvested, processed and stained for flow cytometry analysis. Regulatory T cells were identified by CD4 and FoxP3 expression. Percentage of Foxp3-positive from total CD4-positive cells shows how the anti-CD25 effect is due to the expression of Fcer1g gene. Analysis of *Fcer1g^{-l-}* mice, which do not express any of the activating FcγRs (I, III and IV), demonstrated a complete absence of Treg depletion. Treg elimination by αCD25-r1 in the periphery and αCD25-m2a in the periphery and tumor therefore results from FcγR-mediated ADCC and not blocking of IL-2 binding to CD25. Depletion by αCD25-m2a was not dependent on any individual activatory FcγR, with Treg elimination maintained in both *Fcgr3*^{*-*/*-*} and *Fcgr4*^{*-*/*-*} mice. Thus, depletion of peripheral Treg by αCD25-r1 fails to deplete in the tumor despite high intra-tumoral expression of this receptor. Intra-tumoral Treg depletion is however effectively restored in mice lacking expression of the inhibitory receptor FcγRIIb. In this setting, intra-tumoral Treg depletion is comparable between αCD25-r1 and αCD25-m2a. Therefore, the lack of Treg depletion by αCD25-r1 in the tumor can be explained by its low A/I binding ratio and high intra-tumoral expression of FcγRIIb, which inhibits ADCC mediated by the single activatory receptor engaged by this isotype.

### EXAMPLE 3 - Anti-CD25 therapy synergizes with anti-PD-1, eradicates established tumours and increases survival of tumour-bearing mice

Because of its better efficiency in intra-tumoural Treg depletion, it was hypothesized that αCD25-m2a could have a better therapeutic outcome in the treatment of established tumours. The anti-tumor activity of αCD25-m2a and -r1 against established tumours was evaluated by administering a single dose of αCD25 five days after subcutaneous implantation of MCA205 cells, when tumours were established. The results are provided in Figure 6.

Consistent with the observed lack of capacity to deplete intra-tumoral Treg, a single dose of αCD25 given to mice with established tumours (day 5) resulted in no protection with αCD25-r1. On the other hand, growth delay and long term survival of mice given αCD25-m2a was observed (15.4%). Because of the clinical relevance of agents targeting the co-inhibitory receptor PD-1 as immunotherapeutic target and PD-1 key role in controlling T cell regulation within the tumor microenvironment, we hypothesized that depletion of CD25⁺ Treg cells and PD-1 blockade might be synergistic in combination. In the same model, the combination of αCD25 with PD-1 blockade using anti-PD-1 (αPD-1, clone RMP1-14; at a dose of 100 µg every three days) was tested. αPD-1 as a monotherapy is not effective in the treatment of established MCA205 tumour model and combination with αCD25-r1 did not improve its effect. However, a single dose of αCD25-m2a followed by αPD-1 therapy eradicated established tumours in 78.5% of the mice resulting in long-term survival of more than 100 days. A similar result was observed in MC38 and CT26 tumour models, where αCD25-m2a had a partial therapeutic effect that synergized with αPD-1 therapy, in contrast to combination with αCD25-r1 which failed to deplete tumour-infiltrating Treg in these tumors. Thus, this combined administration allowed an efficient tumour elimination dramatically improved long-term survival of different tumour mice models.

To understand the mechanism of action underlying the synergism with the αCD25-m2a and αPD-1 combination, we evaluated the phenotype and function of tumor-infiltrating lymphocytes (TILs) present in the MCA205 tumour microenvironment at the end of the treatment protocol, 24 hours after the third dose of αPD-1 (Figure 7). Monotherapy with αPD-1 did not impact on Teff proliferation nor on the magnitude of Teff infiltration in the tumor, where we also observed a persisting high frequency of Treg (data not shown), and low ratio of Teff/Treg in keeping with the lack of therapeutic activity. Conversely, intra-tumoral Treg depletion with αCD25-m2a resulted in a higher proportion of proliferating and interferon-γ (IFN-γ)-producing CD4⁺FoxP3⁻ T cells in the tumor, corresponding to a high Teff/Treg ratio and anti-tumor response. This effect was further enhanced in combination with anti-PD-1, which yielded even higher proliferation and a 1.6-fold increase in the number of IFN-γ-producing CD4-positive, FoxP3-negative T cells compared to monotherapy with anti-CD25-m2a. In contrast, the observed lack of Treg depletion with anti-CD25-r1 resulted in no change in Teff proliferation or IFN-γ production, when used as monotherapy or in combination with anti-PD-1.

The data that have been generated with PC61 having either the original mouse IgG1 isotype or the mouse IgG2a isotype that allow efficient Treg depletion suggest the such anti-CD25, alone or in combination of anti-cancer antibodies may be effective at rejecting established tumours, particularly for those tumours requiring efficient intra-tumoural Treg depletion.

As shown above, the administration of a single dose of αCD25-m2a, followed by αPD-1 therapy had a positive effect on both tumour size and mice survival in the MCA205 murine model. This therapeutic effect due to anti-CD25-m2a/anti-PD1 is dependent from the activity of CD8-positive T cell since the further administration of an anti-CD8 antibody brought tumour size and mice survival to the levels observed in untreated animals (Fig. 8). Thus, the MCA205 tumour elimination depends on the impact of αPD-1/αCD25 synergism on both CD8-positive and Treg cell populations, and that overall effector T cell responses are not negatively impacted by a depleting anti-CD25 antibody.

Such a synergy was also observed against the poorly immunogenic B16 melanoma tumour model when αCD25-m2a and αPD-1 were combined with Gvax, a GM-CSF-expressing whole tumour cell vaccine (Fig. 9). In this system, neither Gvax therapy alone nor the combination of Gvax with αPD-1 or αCD25-r1 are able to block tumour growth or to extend survival of tumour-bearing mice. In this setting, only the combination of Gvax with αCD25-m2a (alone or together with αPD-1). Such improved was not observed in any treatment group where αCD25-r1 was administered.

A similar result about the synergism of an immune checkpoint inhibitor with αCD25-m2a was observed in MC38 tumour model both when an αPD-1 (Fig. 10) or an αPD-L1 (Fig. 11) is administered. Also the CT26 tumour models confirmed the therapeutic effect of these combinations (Fig. 12 and 13). Thus, where αCD25-m2a had already a partial therapeutic effect due to the Treg depletion, this advantageous property give the possibility to surprisingly improve the response to therapies based on immune checkpoint inhibitors.

### EXAMPLE 4 - CD25 is highly expressed on Treg infiltrating human tumours and Anti-PD-1 therapy induces infiltration of CD25-expressing Treg in human tumours

CD25 appears an attractive target for Treg depletion and combination immunotherapeutic approaches based on mouse models.. In order to validate CD25 as a possible target for Treg depletion in humans, its expression levels in peripheral blood and tumour-infiltrating lymphocytes were compared using biological samples obtained from ovarian cancer, bladder cancer, melanoma, non-small cell carcinoma (NSCS) and renal cell carcinoma (RCC) patients by flow cytometry and immunohistochemistry (IHC). The number of Treg and CD25 expression in tumour samples from patients with RCC before and after receiving αPD-1 therapy with Pembrolizumab were also quantified. Results are shown in Figures 14 and 15.

Independently of the anatomical location, tumour type or stage, it was observed that CD25 expression in Treg is significantly higher (50-85%) than in CD4+Foxp3- (10-15%) and CD8+ (<5%) T cells. Similar to murine models, the level of CD25 expression, as assessed by MFI, was significantly higher on CD4+FoxP3+ Treg relative to CD4+FoxP3- and CD8+ Teff within all studied tumour subtypes.

These observations were further supported by multiplex immunohistochemistry (IHC). Analysis of melanoma, NSCLC and RCC tumours from the same patient cohorts demonstrated that even in areas of dense CD8-positive, T cell infiltrate, CD25 expression remained restricted to FoxP3-positive cells. Strikingly, this expression profile remained consistent, regardless of tumour subtype, stage, resection site, current or prior therapy and they are in keeping with the data obtained in mouse models.

In addition, in contrast with the high proportion of Treg observed in subcutaneous murine tumours, RCC samples showed a scarce number of Treg in untreated tumours. However, anti-PD-1 therapy resulted in a significant infiltration both by CD8+ T cells and Treg (Foxp3-positive cells). Moreover, data generated from melanoma and RCC samples confirm that CD25 was highly expressed by Foxp3-positive cells, while its expression was minimal in Foxp3-negative, CD8-positive cells.

When CD25 expression is assessed in the context of therapeutic immune modulation. Core biopsies were performed on the same lesion at baseline and following 4 cycles of either Nivolumab or 2 cycles of Pembrolizumab) in patients with advanced kidney cancer and melanoma respectively. Despite systemic immune modulation, CD25 expression remained restricted to FoxP3-positive Treg, even in areas of dense CD8-positive T cell infiltrate evaluated by multiplex IHC.

These findings confirm the translational value of the described pre-clinical data and lend further support to the concept of selective therapeutic targeting of Treg via CD25 in human cancers. Moreover, CD25 expression profiles in human solid cancers in connection with anti-PD1 treatment provides a rationale for the therapeutic combination of anti-human CD25 antibodies having CD25 binding and Fcgamma receptor specificity comparably to those shown for anti-mouse CD25 PC61(IgG2a) with immune checkpoint inhibitors such as a PD-1 antagonist.

### EXAMPLE 5 - Anti-CD25- and anti-PD-L1-based bispecific antibodies and combination of antibodies present efficient Treg depletion and cytokine inducing properties

The previous examples have shown that the Treg depleting, CD25-binding properties of antibodies based on PC61 and with appropriate isotype can be exploited in combination with other anti-cancer compounds such as antibodies targeting immune checkpoint proteins such as a PD-1 antagonist (being an anti-PD-1 or an anti-PD-L1 antibody). These findings suggest the construction of bispecific antibodies combining the two antigen-binding properties and the relevant isotype (e.g. IgG1).

This approach has been validated by using Duobody technology that allows the efficient association of single Heavy and light chain from two distinct monospecific antibodies that are produced separately, within a single heteromeric protein that is named Bs CD25 PD-L1 (Fig. 16A). The binding specificity of this antibody has been validated using two genetically modified human cell lines, each expressing either mouse CD25 or mouse PD-L1, and compared with those of the initial monospecific antibodies (Fig. 16B and C). These cell lines have been tested by flow cytometry, separately or mixed in equivalent amounts, showing that Bs CD25 PD-L1 retains its dual CD25, PD-L1 specificity, even allowing detecting complex of double positive cell complexes that formed by binding of Bs CD25 PD-L1 to both CD25-positive and PD-L1-positive cells at the same time.

The functional properties of BsAb CD25 PD-L1 have been evaluated in vivo by using models of cell interaction and depletion that were used for validating PC61 in previous Examples. The MCA205 model was used for evaluating the impact of the BsAb on effector and regulatory T cells in tumour and LN. In this model, BsAb CD25 PD-L1 can recognize and deplete CD4positive, Foxp3positive regulatory T cells and increase the CD8-positive, Foxp3-positive regulatory T cells ratio in tumours and LN with equivalent efficacy to anti-CD25 (PC61-m2a) or combination of monospecific anti-CD25 and anti-PD-L1 antibodies (Fig. 17 AB). Moreover BsAb CD25 PD-L1 increase the number of Interferon gamma expressing, CD4-positive, CD5-positive cells at a level that is at least similar to the combination of monospecific anti-CD25 and anti-PD-L1, and possibly superior to the one of anti-CD25 m2a antibody alone (Fig. 17C).

The data shows how the use of a PC61-based, Treg depleting, anti-human CD25 antibody for treating cancer can be not only improved by selecting the appropriate isotype but also efficiently combined with other anti-cancer drug, in particular with anti-cancer antibodies that bind to a different cell surface antigen. This approach can be pursued by producing and administering the two products as a novel mixture of monospecific antibodies or as novel bispecific antibodies that are associated and produced in order to maintain the Treg depleting, CD25 binding and other binding properties of the parent monoclonal antibodies.

## Claims

1. An anti-CD25 antibody for use in the treatment of a solid tumour in a human subject, in combination with a PD-1 antagonist, wherein the anti-CD25 antibody is a monoclonal human IgG1 antibody that binds to at least one of FcγRI, FcγRIIc, and FcγRIIIa with higher affinity than it binds to FcγRIIb, and depletes tumour-infiltrating regulatory T cells, and wherein the PD-1 antagonist is an anti-PD-1 antibody or an anti-PD-L1 antibody.

2. An anti-CD25 antibody for use according to claim 1, wherein the anti-CD25 antibody elicits a CDC, ADCC and/or ADCP response.

3. An anti-CD25 antibody for use according to any one of claims 1 to 2 wherein said anti-CD25 antibody is administered to a subject who has an established tumour.

4. An anti-CD25 antibody for use according to any one of claims 1 to 3 wherein said use further comprises the step of identifying a subject who has a solid tumour.

5. A combination of an anti-CD25 antibody and a PD-1 antagonist for use in the treatment of cancer in a human subject, wherein the subject has a solid tumor, wherein the anti-CD25 antibody is a monoclonal human IgG1 antibody that binds to at least one of FcγRI, FcγRIIc, and FcγRIIIa with higher affinity than it binds to FcγRIIb, and depletes tumour-infiltrating regulatory T cells, wherein the anti-CD25 antibody and a PD-1 antagonist are administered simultaneously, separately or sequentially, and wherein the PD-1 antagonist is an anti-PD-1 antibody or an anti-PD-L1 antibody.

6. The combination for use according to claim 5, wherein the anti-CD25 antibody is as defined in claim 2.

## Patentansprüche

1. Anti-CD25-Antikörper zur Verwendung bei der Behandlung eines soliden Tumors in einem humanen Individuum in Kombination mit einem PD-1-Antagonisten, wobei der Anti-CD25-Antikörper ein monoklonaler humaner IgG1-Antikörper ist, der an mindestens einen von FcyRI, FcyRIIc und FcγRIIIa mit höherer Affinität bindet, als er an FcγRIIb bindet, und tumorinfiltrierende regulatorische T-Zellen depletiert und wobei der PD-1-Antagonist ein Anti-PD-1-Antikörper oder ein Anti-PD-L1-Antikörper ist.

2. Anti-CD25-Antikörper zur Verwendung nach Anspruch 1, wobei der Anti-CD25-Antikörper eine CDC-, ADCC- und/oder ADCP-Antwort hervorruft.

3. Anti-CD25-Antikörper zur Verwendung nach einem der Ansprüche 1 bis 2, wobei der Anti-CD25-Antikörper einem Individuum verabreicht wird, das einen etablierten Tumor hat.

4. Anti-CD25-Antikörper zur Verwendung nach einem der Ansprüche 1 bis 3, wobei die Verwendung ferner den Schritt des Identifizierens eines Individuums, das einen soliden Tumor hat, umfasst.

5. Kombination aus einem Anti-CD25-Antikörper und einem PD-1-Antagonisten zur Verwendung bei der Behandlung von Krebs in einem humanen Individuum, wobei das Individuum einen soliden Tumor hat, wobei der Anti-CD25-Antikörper ein monoklonaler humaner IgG1-Antikörper ist, der an mindestens einen von FcyRI, FcyRIIc und FcγRIIIa mit höherer Affinität bindet, als er an FcyRIIb bindet, und tumorinfiltrierende regulatorische T-Zellen depletiert, wobei der Anti-CD25-Antikörper und ein PD-1-Antagonist gleichzeitig, getrennt oder aufeinanderfolgend verabreicht werden und wobei der PD-1-Antagonist ein Anti-PD-1-Antikörper oder ein Anti-PD-L1-Antikörper ist.

6. Kombination zur Verwendung nach Anspruch 5, wobei der Anti-CD25-Antikörper wie in Anspruch 2 definiert ist.

## Revendications

1. Anticorps anti-CD25 pour une utilisation dans le traitement d'une tumeur solide chez un sujet humain, en association avec un antagoniste de PD-1, dans lequel l'anticorps anti-CD25 est un anticorps IgG1 monoclonal humain qui se lie à au moins un parmi FcyRI, FcγRIIc et FcγRIIIa avec une affinité supérieure à celle le liant à FcγRIIb, et réduit les lymphocytes T régulateurs infiltrant les tumeurs, et dans lequel l'antagoniste de PD-1 est un anticorps anti-PD-1 ou un anticorps anti-PD-L1.

2. Anticorps anti-CD25 pour une utilisation selon la revendication 1, dans lequel l'anticorps anti-CD25 déclenche une réponse de CDC, ADCC et/ou ADCP.

3. Anticorps anti-CD25 pour une utilisation selon l'une quelconque des revendications 1 à 2 dans lequel ledit anticorps anti-CD25 est administré à un sujet qui présente une tumeur établie.

4. Anticorps anti-CD25 pour une utilisation selon l'une quelconque des revendications 1 à 3 dans lequel ladite utilisation comprend en outre l'étape d'identification d'un sujet qui présente une tumeur solide.

5. Association d'un anticorps anti-CD25 et d'un antagoniste de PD-1 pour une utilisation dans le traitement d'un cancer chez un sujet humain, dans laquelle le sujet présente une tumeur solide, dans laquelle l'anticorps anti-CD25 est un anticorps IgG1 monoclonal humain qui se lie à au moins un parmi FcyRI, FcγRIIc et FcγRIIIa avec une affinité supérieure à celle le liant à FcγRIIb, et réduit les lymphocytes T régulateurs infiltrant les tumeurs, dans laquelle l'anticorps anti-CD25 et un antagoniste de PD-1 sont administrés simultanément, séparément ou séquentiellement, et dans laquelle l'antagoniste de PD-1 est un anticorps anti-PD-1 ou un anticorps anti-PD-L1.

6. Association pour une utilisation selon la revendication 5, dans laquelle l'anticorps anti-CD25 est tel que défini dans la revendication 2.
